# EUROPEAN PATENT APPLICATION

(11) **EP 3 777 557 A1**
(43) Date of publication of application: **17.02.2021**
(21) Application number: 19775026.8
(22) Date of filing: 26.03.2019
(51) Int. Cl.: A23L 5/00, A23L 7/00, C12N 9/10, C12N 15/09

(54) **METHOD FOR MANUFACTURING STARCH-CONTAINING FOOD**

(30) Priority: 26.03.2018 JP 2018058931
(71) Applicant: AJINOMOTO CO., INC., Chuo-ku Tokyo 104-8315 (JP)
(72) Inventor: AKAMOTO, Kazuto, Kawasaki-shi, Kanagawa 210-8681 (JP); SUGINO, Kazumi, Kawasaki-shi, Kanagawa 210-8681 (JP); YOKOYAMA, Noriko, Kawasaki-shi, Kanagawa 210-8681 (JP)
(74) Representative: Strehl Schübel-Hopf & Partner
(86) International application number: PCT/JP2019/012670
(87) International publication number: WO 2019/189065

(57) **Abstract**

The present invention provides a production method of a starch-containing food, including reacting actinomycete-derived amylomaltase with starch in the raw material.

SEQUENCE LISTING

<110>  AJINOMOTO CO., INC.

<120>  Method for producing a food containing starch

<130>  092881

<150>  JP2018-058931
<151>  2018-03-26

<160>  6

<170>  PatentIn version 3.5

<210>  1
<211>  706
<212>  PRT
<213>  Corynebacterium glutamicum

<400>  1

Met Thr Ala Arg Arg Phe Leu Asn Glu Leu Ala Asp Leu Tyr Gly Val
1               5                   10                  15

Ala Thr Ser Tyr Thr Asp Tyr Lys Gly Ala His Ile Glu Val Ser Asp
            20                  25                  30

Asp Thr Leu Val Lys Ile Leu Arg Ala Leu Gly Val Asn Leu Asp Thr
        35                  40                  45

Ser Asn Leu Pro Asn Asp Asp Ala Ile Gln Arg Gln Ile Ala Leu Phe
    50                  55                  60

His Asp Arg Glu Phe Thr Arg Pro Leu Pro Pro Ser Val Val Ala Val
65                  70                  75                  80

Glu Gly Asp Glu Leu Val Phe Pro Val His Val His Asp Gly Ser Pro
                85                  90                  95

Ala Asp Val His Ile Glu Leu Glu Asp Gly Thr Gln Arg Asp Val Ser
            100                 105                 110

Gln Val Glu Asn Trp Thr Ala Pro Arg Glu Ile Asp Gly Ile Arg Trp
        115                 120                 125

Gly Glu Ala Ser Phe Lys Ile Pro Gly Asp Leu Pro Leu Gly Trp His
    130                 135                 140

Lys Leu His Leu Lys Ser Asn Glu Arg Ser Ala Glu Cys Gly Leu Ile
145                 150                 155                 160

Ile Thr Pro Ala Arg Leu Ser Thr Ala Asp Lys Tyr Leu Asp Ser Pro

                165                 170                 175

Arg Ser Gly Val Met Ala Gln Ile Tyr Ser Val Arg Ser Thr Leu Ser
            180                 185                 190

Trp Gly Met Gly Asp Phe Asn Asp Leu Gly Asn Leu Ala Ser Val Val
        195                 200                 205

Ala Gln Asp Gly Ala Asp Phe Leu Leu Ile Asn Pro Met His Ala Ala
    210                 215                 220

Glu Pro Leu Pro Pro Thr Glu Asp Ser Pro Tyr Leu Pro Thr Thr Arg
225                 230                 235                 240

Arg Phe Ile Asn Pro Ile Tyr Ile Arg Val Glu Asp Ile Pro Glu Phe
                245                 250                 255

Asn Gln Leu Glu Ile Asp Leu Arg Asp Asp Ile Ala Glu Met Ala Ala
            260                 265                 270

Glu Phe Arg Glu Arg Asn Leu Thr Ser Asp Ile Ile Glu Arg Asn Asp
        275                 280                 285

Val Tyr Ala Ala Lys Leu Gln Val Leu Arg Ala Ile Phe Glu Met Pro
    290                 295                 300

Arg Ser Ser Glu Arg Glu Ala Asn Phe Val Ser Phe Val Gln Arg Glu
305                 310                 315                 320

Gly Gln Gly Leu Ile Asp Phe Ala Thr Trp Cys Ala Asp Arg Glu Thr
                325                 330                 335

Ala Gln Ser Glu Ser Val His Gly Thr Glu Pro Asp Arg Asp Glu Leu
            340                 345                 350

Thr Met Phe Tyr Met Trp Leu Gln Trp Leu Cys Asp Glu Gln Leu Ala
        355                 360                 365

Ala Ala Gln Lys Arg Ala Val Asp Ala Gly Met Ser Ile Gly Ile Met
    370                 375                 380

Ala Asp Leu Ala Val Gly Val His Pro Gly Gly Ala Asp Ala Gln Asn
385                 390                 395                 400

Leu Ser His Val Leu Ala Pro Asp Ala Ser Val Gly Ala Pro Pro Asp
                405                 410                 415

Gly Tyr Asn Gln Gln Gly Gln Asp Trp Ser Gln Pro Pro Trp His Pro
            420                 425                 430

Val Arg Leu Ala Glu Glu Gly Tyr Ile Pro Trp Arg Asn Leu Leu Arg
        435                 440                 445

Thr Val Leu Arg His Ser Gly Gly Ile Arg Val Asp His Val Leu Gly
    450                 455                 460

Leu Phe Arg Leu Phe Val Met Pro Arg Met Gln Ser Pro Ala Thr Gly
465                 470                 475                 480

Thr Tyr Ile Arg Phe Asp His Asn Ala Leu Val Gly Ile Leu Ala Leu
                485                 490                 495

Glu Ala Glu Leu Ala Gly Ala Val Val Ile Gly Glu Asp Leu Gly Thr
            500                 505                 510

Phe Glu Pro Trp Val Gln Asp Ala Leu Ala Gln Arg Gly Ile Met Gly
        515                 520                 525

Thr Ser Ile Leu Trp Phe Glu His Ser Pro Ser Gln Pro Gly Pro Arg
    530                 535                 540

Arg Gln Glu Glu Tyr Arg Pro Leu Ala Leu Thr Thr Val Thr Thr His
545                 550                 555                 560

Asp Leu Pro Pro Thr Ala Gly Tyr Leu Glu Gly Glu His Ile Ala Leu
                565                 570                 575

Arg Glu Arg Leu Gly Val Leu Asn Thr Asp Pro Ala Ala Glu Leu Ala
            580                 585                 590

Glu Asp Leu Gln Trp Gln Ala Glu Ile Leu Asp Val Ala Ala Ser Ala
        595                 600                 605

Asn Ala Leu Pro Ala Arg Glu Tyr Val Gly Leu Glu Arg Asp Gln Arg
    610                 615                 620

Gly Glu Leu Ala Glu Leu Leu Glu Gly Leu His Thr Phe Val Ala Lys
625                 630                 635                 640

Thr Pro Ser Ala Leu Thr Cys Val Cys Leu Val Asp Met Val Gly Glu
                645                 650                 655

Lys Arg Ala Gln Asn Gln Pro Gly Thr Thr Arg Asp Met Tyr Pro Asn
            660                 665                 670

Trp Cys Ile Pro Leu Cys Asp Ser Glu Gly Asn Ser Val Leu Ile Glu
        675                 680                 685

Ser Leu Arg Glu Asn Glu Leu Tyr His Arg Val Ala Lys Ala Ser Lys
    690                 695                 700

Arg Asp
705

<210> 2
<211> 2121
<212> DNA
<213> Corynebacterium glutamicum

<400> 2
gtgactgctc gcagattttt gaatgaactc gccgatctct acggcgtagc aacttcctac 60

actgattaca aaggtgccca tattgaggtc agcgatgaca cattagtgaa aatcctgcgt 120

gctctgggtg tgaatttaga tacaagcaac ctccccaacg atgacgctat ccaacgccaa 180

attgccctct tccatgatcg agagttcact cgcccactgc ctccatcggt ggttgcagtt 240

gaaggtgatg aactagtttt cccggtgcat gtgcacgacg gttcccctgc agatgtccac 300

atcgaattgg aagacggcac gcagcgggat gtttctcagg tggaaaactg gacagcgcca 360

cgggaaattg atgggattag gtggggcgag gcatcgttta agattcctgg tgatctcccc 420

ttgggttggc acaagcttca ccttaaatcc aatgaacgct cagctgagtg cggtttgatc 480

atcaccccgg ctcgtctgtc tactgctgat aagtatcttg attcccctcg cagtggtgtc 540

atggcgcaga tctactctgt gcgttccacg ttgtcgtggg gcatgggtga tttcaatgat 600

ttaggaaact tggcaagtgt ggttgcccag gatggagcag acttcctgct catcaacccc 660

atgcacgctg cagagccgct gcctcctact gaggactctc cttatctgcc cacaaccagg 720

cgctttatca acccgatcta cattcgggta gaagatattc cggagtttaa tcagcttgag 780

attgatctac gcgatgatat cgcagagatg gctgcggaat tccgcgaacg caatctgacc 840

tcagacatca ttgagcgcaa tgacgtctac gctgcaaagc ttcaagtgct gcgcgccatt 900

tttgaaatgc ctcgttccag cgaacgtgaa gccaactttg tctccttcgt gcaacgggaa 960

ggccaaggtc ttattgattt cgccacctgg tgcgcggacc gcgaaactgc acagtctgaa 1020

tctgtccacg gaactgagcc agaccgcgat gagctgacca tgttctacat gtggttgcag 1080

tggctatgtg atgagcagct ggcggcagct caaaagcgcg ctgtcgatgc cggaatgtcg 1140

atcggcatca tggcagacct ggcagttggt gtgcatccag gtggtgctga tgcccagaac 1200

ctcagccacg tacttgctcc ggatgcgtca gtgggcgccc caccagatgg atacaaccag 1260

cagggccaag actggtccca gccaccatgg catccagtgc gtcttgcaga ggaaggctac 1320

attccgtggc gtaatctgct gcgcactgtg ctgcgtcact ccggcggaat ccgcgtggac 1380

cacgttcttg gtttgttcag gctctttgtc atgccacgca tgcaatcccc tgctacgggc 1440

acctatatcc gcttcgacca taatgcgttg gtaggcattc tagccctaga agcagaactc 1500

gcaggcgccg ttgtcattgg tgaagatctg ggaacgtttg agccttgggt acaagatgca 1560

ttggctcagc gtggcatcat gggcacctcg atcctatggt tcgagcattc cccaagccag 1620

ccgggtcctc gccgccagga agagtatcgt ccgctggcct tgaccactgt gaccactcat 1680

gatctccctc cgactgctgg ttatttggag ggcgagcaca ttgctcttcg tgagcgattg 1740

ggggtgctca acactgatcc tgctgcagaa ctcgctgagg atctgcagtg gcaagcggag 1800

atccttgatg tcgcagcatc tgccaacgca ttgccagccc gggaatacgt gggactcgaa 1860

cgcgatcagc gcggtgagtt ggctgagctg ttggaaggcc tgcacacttt cgttgcgaaa 1920

accccttcag cactgacctg tgtctgcttg gtagacatgg tcggtgaaaa gcgggcacag 1980

aatcagccgg gcacaacgag ggatatgtat cccaactggt gtatcccact gtgtgacagc 2040

gaaggcaact ccgtgctcat tgaatcgctg cgtgaaaatg agctgtatca ccgtgtggca 2100

aaggcaagca agcgagatta g 2121

<210> 3
<211> 740
<212> PRT
<213> Streptomyces avermitilis

<400> 3

Met Pro Pro Ala Glu Thr Gly Glu Ser Glu Thr Asp Glu Pro Gly Glu
1               5                   10                  15

Ala Asp Arg Ala His Ala Ala Asp Gly Ile His Gly Ala Asp Glu Thr
            20                  25                  30

Asp Gly Ala His Glu Ala His Gly Val Gly Glu Ala Pro Leu Ala Arg
        35                  40                  45

Leu Ala Ala Leu His Gly Val Ala Thr Ser Tyr Ser Pro Ser Pro Gly
    50                  55                  60

Arg Thr Val Ala Ala Ser Asp Ala Ala Val Thr Thr Ala Leu Ala Ala
65                  70                  75                  80

Leu Gly Val Asp Ala Gly Thr Pro Asp Ala Val His Arg Ala Leu Ala
                85                  90                  95

Ala Arg Glu Thr Glu Leu Arg Arg Arg Leu Leu Pro Pro Thr Val Val
            100                 105                 110

Cys Trp Gly Glu Gln Pro Pro Ala Ala Leu Thr Asp Leu Pro Asp Gly
        115                 120                 125

Thr Arg Leu Arg Ile Glu Thr Glu Gln Gly Glu Thr Arg Ala Ser Ala
    130                 135                 140

Glu Gln Leu Pro Pro Gly Val His Ala Leu His Ile Thr Ala Pro Asp
145                 150                 155                 160

Gly Arg Ser Ala Asp Ala His Leu Val Val Ala Pro Ala Arg Leu Ser
                165                 170                 175

Ala Pro Pro Gly Arg Ala Tyr Gly Leu Leu Val Gln Leu Tyr Ser Leu
            180                 185                 190

Leu Ser Gln Arg Ser Trp Gly Met Gly Asp Leu Gly Asp Leu Ala Glu
        195                 200                 205

Leu Ala Ala Trp Ala Gly Arg Ala Leu Gly Ala Gly Phe Val Gln Leu
    210                 215                 220

Asn Pro Leu His Val Ala Val Pro Gly Asp Pro Thr Asp Pro Ser Pro
225                 230                 235                 240

Tyr Arg Pro Ser Ser Arg Arg Tyr Pro Asp Pro Val His Leu Arg Val
                245                 250                 255

Glu Asp Val Pro Glu Phe Ala Tyr Val Asp Asp Pro Gly Pro Val Arg
            260                 265                 270

Thr Leu Leu Asp Arg Ala Ala Arg Leu Arg Glu Ser Val Leu Arg Lys
        275                 280                 285

Gly Ala Leu Ile Asp Arg Asp Ala Val Trp Glu Leu Lys Arg Glu Ala
    290                 295                 300

Leu Glu Arg Val His Ala Val Pro Leu Gly Pro Gly Arg Arg Ala Ala
305                 310                 315                 320

Tyr Val Asp Phe Leu Ala Glu Glu Gly Glu Ala Leu Glu Asp His Ala
                325                 330                 335

Thr Trp Cys Ala Leu Ala Glu Val Tyr Gly Ala Asp Gly His Gly Trp
            340                 345                 350

Pro Ala Gly Leu Arg Asp Pro Arg Ser Ala Glu Thr Ala Arg Ala Arg
        355                 360                 365

Gly Glu Leu Met Asp Arg Val Asp Phe His Ala Trp Leu Ala Trp Leu
    370                 375                 380

Thr Asp Glu Gln Leu Ala Ala Ala Gln Arg Ala Ala Arg Asp Ala Gly
385                 390                 395                 400

Met Ala Val Gly Leu Val His Asp Leu Ala Val Gly Val His Pro Val
                405                 410                 415

Gly Ala Asp Ala Trp Ala Gln Gln Glu Tyr Phe Ala Ala Gly Met Ser
            420                 425                 430

Val Gly Ala Pro Pro Asp Ala Phe Asn Ala Leu Gly Gln Asp Trp Gly
        435                 440                 445

Leu Pro Pro Trp Arg Pro Asp Arg Leu Ala Glu Ser Gly Tyr Ala Pro
    450                 455                 460

Tyr Arg Arg Leu Leu Arg Ala Leu Phe Arg Tyr Ala Gly Ala Leu Arg
465                 470                 475                 480

Ile Asp His Val Met Gly Leu Phe Arg Leu Trp Trp Val Pro Gln Gly
                485                 490                 495

Arg Pro Pro Thr Glu Gly Thr Tyr Val Arg Tyr Asp Ala Glu Ala Met
            500                 505                 510

Leu Ala Ile Leu Val Leu Glu Ala Ser Arg Thr Gly Ala Leu Val Ile
        515                 520                 525

Gly Glu Asp Leu Gly Thr Val Glu Pro Gly Val Arg Glu Thr Leu His
    530                 535                 540

Ala Arg Gly Val Leu Gly Thr Ser Val Leu Trp Phe Glu Arg Asp Trp
545                 550                 555                 560

Glu Gly Asp Gly Leu Pro Leu Pro Pro Glu Arg Trp Arg Ala Asp Cys
                565                 570                 575

Leu Ala Thr Ala Thr Thr His Asp Leu Pro Pro Thr Ala Ala Arg Leu
            580                 585                 590

Thr Gly Ala His Val Glu Leu Arg Asp Arg Leu Gly Leu Leu Ala Arg
        595                 600                 605

Pro Leu Glu Glu Glu Arg Ala Ala Ala Ala Ala Asp Thr Ala Glu Trp

610                 615                 620

Leu Ala Leu Leu Ala Arg Leu Gly Leu Leu Lys Gly Ala Ser Gly Gly
625                 630                 635                 640

Ile Cys Ser Val Ser Glu Glu Ala Glu Ile Gln Ala Val His Ala Phe
                645                 650                 655

Leu Leu Arg Thr Pro Ala Arg Leu Val Gly Leu Trp Leu Pro Asp Ala
            660                 665                 670

Val Gly Asp Arg Arg Pro Gln Asn Leu Pro Gly Thr Trp Asp Gln Tyr
        675                 680                 685

Pro Asn Trp Arg Leu Pro Ile Ala Asp Ala Arg Gly Arg Pro Val Thr
    690                 695                 700

Leu Glu Glu Leu Ala Ala Ala Pro Arg Leu His Ala Leu Val Asp Val
705                 710                 715                 720

Leu Arg Gly Leu Pro Ala Gly Gly Gly Ala Pro Gly Gly Ala Arg Cys
                725                 730                 735

Gly Glu Arg Gly
            740

<210>  4
<211>  703
<212>  PRT
<213>  Streptomyces cinnamoneus

<400>  4

Met Asp Arg Ala Arg Leu Ala Ala Ala His Gly Val Ala Thr Ser Tyr
1               5                   10                  15

Glu Ser Ala Pro Gly Arg Ser Val Pro Val Pro Glu Asp Thr Val Val
            20                  25                  30

Ala Val Leu Ala Ala Leu Gly Val Asp Ala Ser Thr Pro Arg Ala Val
        35                  40                  45

Arg Asp Ala Leu Glu Ala Arg Glu Arg Ala Glu Arg Thr Arg Leu Leu
    50                  55                  60

Pro Pro Thr Val Val Leu Arg Pro Gly Gly Glu Arg Leu Pro Ala Leu
65                  70                  75                  80

Pro Glu Gly Thr Thr Leu Arg Val Asp Thr Glu Asp Gly Arg Ala Leu

                85                  90                  95

Ala Trp Asp Gly Ala Ala Pro Leu Pro Leu Gly Gly His Val Leu Arg
            100                 105                 110

Ala His Ala Pro Asp Gly Arg Ser Ala Ser Ala Pro Leu Ile Val Ala
        115                 120                 125

Pro Gly Arg Leu Pro Ala Pro Pro Glu Arg Ala His Gly Phe Leu Val
    130                 135                 140

Gln Leu Tyr Ser Leu Leu Ser Ala Arg Ser Trp Gly Met Gly Asp Leu
145                 150                 155                 160

Ala Asp Leu Ala Glu Leu Ala Ala Trp Ser Gly Arg Ala Phe Gly Ser
                165                 170                 175

Asp Phe Val Gln Ile Asn Pro Leu His Ala Ala Val Pro Gly Pro Pro
            180                 185                 190

Thr Asp Pro Ser Pro Tyr Arg Pro Ser Ser Arg Arg Phe Pro Asp Pro
        195                 200                 205

Val His Leu Arg Val Thr Glu Val Pro Glu Phe Ala His Leu Ser Gly
    210                 215                 220

Glu Ala Arg Ala Glu Ala Asp Arg Leu Leu Ala Arg Ala Ala Gly Leu
225                 230                 235                 240

Arg Asp Ala Val Leu Arg Asp Gly Ala Leu Ile Asp Arg Asp Ala Val
                245                 250                 255

Trp Glu Leu Lys Arg Arg Ala Leu Glu Leu Val Arg Ala Val Pro Leu
            260                 265                 270

Gly Pro Gly Arg Arg Ala Ala Tyr Asp Ala Phe Leu Ala Glu Arg Gly
        275                 280                 285

Gln Ala Leu Asp Asp His Ser Thr Trp Cys Ala Leu Ala Glu Val His
    290                 295                 300

Gly Pro Asp Trp Arg Ala Trp Pro Gly Ala Leu Lys Asp Pro Arg Ser
305                 310                 315                 320

Ala Arg Thr Ala Arg Ala Arg Gly Glu Leu Leu Asp Arg Val Asp Phe
                325                 330                 335

His Cys Trp Leu Ala Trp Leu Thr Asp Gly Gln Leu Ala Ala Ala Gln
        340                 345                 350

Arg Ala Ala Arg Glu Ala Gly Met Arg Ile Gly Leu Val His Asp Leu
    355                 360                 365

Ala Val Gly Val His Pro Ser Gly Ala Asp Thr Trp Ala Gln Gln Asp
    370                 375                 380

Ala Phe Ala Ala Gly Met Ser Val Gly Ala Pro Pro Asp Ala Phe Ser
385                 390                 395                 400

Pro Arg Gly Gln Asp Trp Gly Leu Pro Pro Trp Arg Pro Asp Ala Leu
                405                 410                 415

Ala Ala Thr Gly His Ala Pro Tyr Arg Glu Leu Leu Arg Ala Leu Phe
        420                 425                 430

Arg His Ala Gly Ala Leu Arg Val Asp His Val Met Gly Leu Phe Arg
    435                 440                 445

Leu Trp Trp Val Pro Gln Gly Cys Glu Pro Gly Arg Gly Thr Tyr Val
    450                 455                 460

Arg Tyr Asp Ala Glu Ala Met Leu Gly Val Leu Ala Leu Glu Ala His
465                 470                 475                 480

Arg Ala Gly Ala Val Val Val Gly Glu Asp Leu Gly Thr Val Glu Pro
                485                 490                 495

Gly Val Arg Glu Ala Leu Ala Glu Arg Gly Val Leu Gly Thr Ser Val
        500                 505                 510

Leu Trp Phe Glu Arg Ser Tyr Gly Thr Gly Glu Lys Gly Glu Glu Gly
    515                 520                 525

Gly Asp Glu Pro Leu Pro Pro Glu Arg Trp Arg Pro Gly Ala Leu Ala
    530                 535                 540

Thr Ala Thr Thr His Asp Leu Pro Ser Thr Ala Ala Arg Leu Thr Gly
545                 550                 555                 560

Asp His Val Ala Leu Arg His Arg Leu Gly Leu Leu Thr Arg Pro Leu
                565                 570                 575

Ala Glu Glu Gln Arg Glu Asp Ala Ala Ala Val Ala Gln Trp Leu Ala
        580                 585                 590

Leu Phe Gly Arg Leu Gly Leu Leu Pro Glu Gly Pro Gly Asp Glu Glu
        595                 600                 605

Ala Ala Val Arg Ala Val His Arg Phe Leu Leu Leu Thr Pro Ala Arg
    610                 615                 620

Leu Ile Gly Val Trp Leu Pro Asp Ala Val Gly Asp Arg Arg Pro Gln
625                 630                 635                 640

Asn Leu Pro Gly Thr Trp Asp Gln Tyr Pro Asn Trp Arg Leu Pro Leu
                645                 650                 655

Ala Asp Ser Ala Gly Arg Pro Leu Ser Leu Glu Glu Leu Ala Ala Ser
            660                 665                 670

Pro Arg Leu His Ala Leu Met Glu Glu Val Arg Gly Pro Gly Asp Ala
        675                 680                 685

Asp Arg Pro Pro Arg Thr Arg Ala Ala Arg Gly Gly Pro Ile Arg
    690                 695                 700

<210>  5
<211>  839
<212>  PRT
<213>  Streptomyces griseus

<400>  5

Met Gly Leu Ser Arg Leu Ala Ala Leu His Gly Val Ala Thr Ser Tyr
1               5                   10                  15

Ser Pro Ser Pro Asp Val Thr Val Ser Val Pro Asp Asp Thr Val Ile
            20                  25                  30

Ala Val Leu Ala Ala Leu Gly Val Asp Ala Gly Thr Pro Ala Asp Val
        35                  40                  45

Arg Lys Cys Leu Val Ala Ala Glu Ser Arg Ser Arg Leu Leu Pro Pro
    50                  55                  60

Thr Val Val Val Trp Ala Gly Glu Pro Leu Pro Pro Ser Leu Ala Gly
65                  70                  75                  80

Leu Pro Ser Gly Ser Thr Val Thr Val Glu Pro Glu Pro Ala Asp Thr
                85                  90                  95

Pro Gly Pro Ser Gly Arg Pro Pro Ala Pro Leu Ser Met Arg Ala Arg
            100                 105                 110

Ala Ser Ala Pro Ala Pro Pro Val Pro Pro Pro Pro Gly Ala Ala Ala
        115                 120                 125

Ala Pro Leu Thr Pro Pro Gly Thr Ala Ala Thr Thr Thr Ala Pro Val
    130                 135                 140

Ala Ala Ala Ala Ala Ala Pro Pro Ala Thr Glu Ala Ala Ala Pro Ala
145                 150                 155                 160

Ala Val Ala Val Pro Pro Val Thr Gln Ala Gly Asn Ala Asp Ser Ala
                165                 170                 175

Pro Ala Val Thr Pro Val Ala Gly Thr Ala Thr Ala Ser Ala Ser Pro
            180                 185                 190

Ala Ala Ser Ala Ser Ala Ser Ala Ser Pro Ser Ala Thr Ala Ser Ala
        195                 200                 205

Pro Ala Thr Ala Ser Ala Pro Ala Thr Ala Ser Ala Pro Ala Thr Ala
    210                 215                 220

Leu Pro Ser Ala Ser Ala Pro His Thr His Pro Arg Arg Ala Asp Glu
225                 230                 235                 240

Ala Pro Val Val Pro Ala Ala Val Pro Ala Trp Trp Ile Pro Pro Pro
                245                 250                 255

Pro Gly Val His Arg Val His Val Arg Thr Pro Asp His Arg Arg Ala
            260                 265                 270

Thr Ala Thr Leu Val Val Ala Pro Ala Arg Val Pro Gln Pro Pro Glu
        275                 280                 285

Arg Ser His Gly Phe Leu Val Gln Leu Tyr Ser Leu Leu Ser Ala Arg
    290                 295                 300

Ser Trp Gly Met Gly Asp Leu Gly Asp Leu Ala Asp Leu Ala Ala Trp
305                 310                 315                 320

Ala Gly Arg Thr Leu Gly Ser Gly Phe Val Gln Val Asn Pro Leu His
                325                 330                 335

Ala Ala Val Pro Gly Arg Pro Thr Asp Pro Ser Pro Tyr Arg Pro Ser
            340                 345                 350

Ser Arg Arg Phe Pro Asp Pro Val His Leu Arg Val Glu Ser Val Pro
        355                 360                 365

Glu Tyr Gly His Val Arg Asp Arg Ala Ala Leu Asp Asp Leu Arg Gln
    370                 375                 380

Gly Ala Asp Ala Leu Ser Glu Ala Val Leu Asn Lys Gly Ala Leu Ile
385                 390                 395                 400

Asp Arg Asp Ala Val Trp Glu Leu Lys Arg Gln Ala Leu Glu Leu Val
                405                 410                 415

Val Glu Val Pro Leu Thr Pro Gly Arg Arg Ala Ala Tyr Cys Asp Phe
            420                 425                 430

Leu Ala Glu Gln Gly Gln Ala Leu Glu Asp His Ala Leu Trp Cys Ala
        435                 440                 445

Leu Ala Glu Val His Gly Pro Asp Trp Arg Thr Trp Pro Glu Ala Leu
    450                 455                 460

Arg Asp Pro Arg Ser Ser Gly Thr Ala Arg Ala Arg Ser Glu Leu Leu
465                 470                 475                 480

Asp Arg Val Asp Phe His Cys Arg Leu Ala Trp Leu Thr Ala Asp Gln
                485                 490                 495

Leu Ala Ala Ala Gln Arg Ala Ala Glu Glu Ala Gly Met Ser Val Gly
            500                 505                 510

Ile Val His Asp Leu Ala Val Gly Val His Pro Ala Gly Ala Asp Thr
        515                 520                 525

Trp Ala Gln Gln Glu Ala Phe Ala His Gly Met Ser Val Gly Ala Pro
    530                 535                 540

Pro Asp Ala Phe Asn Ala Arg Gly Gln Asp Trp Gly Leu Pro Pro Trp
545                 550                 555                 560

Arg Pro Asp Thr Leu Ala Ala Thr Gly Tyr Ala Ala Tyr Arg Gly Leu
                565                 570                 575

Leu Arg Gly Leu Leu Ala Gly Ala Gly Ala Leu Arg Ile Asp His Val
            580                 585                 590

Met Gly Leu Phe Arg Leu Trp Trp Val Pro Glu Gly Ser Pro Pro Thr
        595                 600                 605

Asp Gly Thr Tyr Val Ala His Asp Ala Glu Ala Met Leu Ala Val Leu

610                 615                 620

Val Leu Glu Ala His Arg Ala Gly Thr Val Val Val Gly Glu Asp Leu
625                 630                 635                 640

Gly Thr Val Ala Pro Gly Val Arg Glu Ala Leu Ala Arg Arg Gly Val
                645                 650                 655

Leu Gly Thr Ser Val Leu Trp Phe Glu Arg Asp Trp Asp Gly Asp Gly
            660                 665                 670

Arg Pro Ile Ala Pro Glu Lys Trp Arg Arg Asp Cys Leu Ala Thr Ala
        675                 680                 685

Thr Thr His Asp Leu Pro Ser Thr Ala Ala Arg Leu Thr Gly Asp His
    690                 695                 700

Val Thr Leu Arg His Arg Leu Gly Leu Leu Thr Arg Ser Leu Glu Glu
705                 710                 715                 720

Glu Leu Thr Ala Asp Leu Thr Asp Thr Ala Glu Trp Leu Ala Leu Leu
                725                 730                 735

Ala Arg Leu Arg Met Leu Pro Glu Gly Asp Gly Asp Glu Glu Ala Ala
            740                 745                 750

Val Arg Ala Val His Arg Phe Leu Leu Arg Thr Pro Ala Arg Met Thr
        755                 760                 765

Gly Val Trp Leu Pro Asp Thr Val Gly Asp Arg Arg Pro Gln Asn Leu
    770                 775                 780

Pro Gly Thr Trp Asp Gln Tyr Pro Asn Trp Arg Leu Pro Ile Ala Asp
785                 790                 795                 800

Ala Glu Gly His Pro Val Thr Leu Glu Glu Ile Thr Ala Ser Pro Arg
                805                 810                 815

Leu His Ala Leu Met Asp Val Leu Arg Pro Arg Lys Pro His Thr Ala
            820                 825                 830

Pro Pro Gly Glu Arg Arg Pro
        835

<210>  6
<211>  776
<212>  PRT
<213>  Streptomyces violaceoruber

<400> 6

Met Gly Leu Ser Arg Leu Ala Ala Leu His Gly Val Ala Thr Ser Tyr
1               5                   10                  15

Ser Pro Ser Pro Asp Ala Thr Val Ser Val Pro Asp Asp Thr Val Ile
            20                  25                  30

Ala Val Leu Ala Ala Leu Gly Val Asp Ala Gly Thr Pro Glu Asp Val
        35                  40                  45

Arg Arg Ser Leu Val Ala Ala Glu Ser Arg Ser Arg Leu Leu Pro Pro
    50                  55                  60

Thr Val Val Val Trp Ala Gly Glu Pro Leu Pro Ala Ala Leu Ala Ser
65                  70                  75                  80

Leu Pro Ser Gly Ser Thr Val Thr Val Glu Pro Glu Ala Ser Gly Pro
                85                  90                  95

Pro Gly Arg His Pro Ala Gly Arg Pro Pro Ala Pro Leu Ser Met Arg
            100                 105                 110

Ser Arg Ala Ala Ala Pro Ala Pro Ala Val Thr Arg Pro Ala Ala Gln
        115                 120                 125

Ala Ala Pro Ala Thr Ala Gly Thr Asp Gly Ala Pro Ala Pro Gly Ala
    130                 135                 140

Leu Ala Thr Ala Ala Ala Leu Val Pro Glu Ala Ala Ala Ala Glu Pro
145                 150                 155                 160

Ser Ala Ala Glu Ala Leu Ala Gly Val Gly Ala Ala Gly Ala Pro Ala
                165                 170                 175

Arg Glu Ala Pro Ala Gly Thr Gly Val Pro Ala Trp Trp Val Pro Pro
            180                 185                 190

Pro His Gly Val His Arg Ile His Val Arg Thr Pro Asp His Arg Arg
        195                 200                 205

Ala Ser Ala Thr Leu Ile Ala Ala Pro Ala Arg Val Pro Gln Pro Ala
    210                 215                 220

Glu Arg Thr His Gly Phe Leu Val Gln Leu Tyr Ser Leu Leu Ser Ala
225                 230                 235                 240

Arg Ser Trp Gly Met Gly Asp Leu Gly Asp Leu Ala Asp Leu Ala Ala
                245                 250                 255

Trp Ala Gly Arg Thr Leu Gly Ser Gly Phe Val Gln Val Asn Pro Leu
            260                 265                 270

His Ala Ala Val Pro Gly Arg Pro Thr Asp Pro Ser Pro Tyr Arg Pro
        275                 280                 285

Ser Ser Arg Arg Phe Pro Asp Pro Val His Leu Arg Val Glu Ser Ile
    290                 295                 300

Pro Glu Tyr Gly His Ile Arg Asp Arg Ala Thr Leu Asp Asp Leu Arg
305                 310                 315                 320

Gln Asp Ala Ala Ala Leu Ser Glu Ala Val Leu Asn Lys Gly Ala Leu
                325                 330                 335

Ile Asp Arg Asp Ala Val Trp Glu Leu Lys Arg Gln Ala Leu Glu Leu
            340                 345                 350

Val Val Arg Val Pro Leu Thr Pro Gly Arg Arg Ala Ala Tyr Cys Asp
        355                 360                 365

Phe Leu Ala Glu Gln Gly Gln Pro Leu Glu Asp His Ala Leu Trp Cys
    370                 375                 380

Ala Leu Ala Glu Val His Gly Pro Asp Trp His Thr Trp Pro Glu Ala
385                 390                 395                 400

Leu Arg Asp Pro Arg Ser Pro Gly Ala Ala Arg Ala Arg Ser Asp Leu
                405                 410                 415

Leu Asp Arg Val Asp Phe His Cys Arg Leu Ala Trp Leu Thr Ala Cys
            420                 425                 430

Gln Leu Ala Glu Ala Gln Arg Ala Ala Glu Asp Ala Gly Met Gly Val
        435                 440                 445

Gly Val Val His Asp Leu Ala Val Gly Val His Pro Ala Gly Ala Asp
    450                 455                 460

Thr Trp Ala Gln Gln Asp Ala Phe Ala Arg Gly Met Ser Val Gly Ala
465                 470                 475                 480

Pro Pro Asp Ala Phe Asn Ala Arg Gly Gln Asp Trp Gly Leu Pro Pro
                485                 490                 495

Trp Arg Pro Asp Thr Leu Ala Ala Thr Gly Tyr Ala Ala Tyr Arg Asp
            500                 505                 510

Leu Leu Arg Ala Arg Leu Ala His Ala Gly Ala Leu Arg Ile Asp His
        515                 520                 525

Val Met Gly Leu Phe Arg Leu Trp Trp Val Pro Glu Gly Arg Pro Pro
    530                 535                 540

Thr Asp Gly Thr Tyr Val Ala Tyr Asp Ala Glu Ala Met Leu Ala Val
545                 550                 555                 560

Leu Val Leu Glu Ala His Arg Ala Gly Thr Ala Val Val Gly Glu Asp
                565                 570                 575

Leu Gly Thr Val Glu Pro Gly Val Arg Glu Ala Leu Ala Arg Arg Gly
            580                 585                 590

Val Leu Gly Thr Ser Val Leu Trp Phe Glu Arg Asp Trp Glu Gly Asp
        595                 600                 605

Gly Arg Pro Ile Ala Pro Glu Lys Trp Arg Arg Ala Cys Leu Ala Thr
    610                 615                 620

Ala Thr Thr His Asp Leu Pro Ser Thr Ala Ala Arg Leu Thr Gly Asp
625                 630                 635                 640

His Val Thr Leu Arg His Arg Leu Gly Leu Leu Thr Arg Ser Leu Glu
                645                 650                 655

Glu Glu Leu Thr Asp Asp Val Thr Asp Thr Ala Glu Trp Leu Ala Leu
            660                 665                 670

Leu Ala Arg Leu Arg Met Leu Pro Glu Gly Asp Gly Asp Glu Glu Ala
        675                 680                 685

Ala Val Arg Ala Val His Arg Phe Leu Leu Arg Thr Pro Ala Leu Leu
    690                 695                 700

Thr Gly Val Trp Leu Pro Asp Thr Val Gly Asp Arg Arg Pro Gln Asn
705                 710                 715                 720

Leu Pro Gly Thr Trp Asp Gln Tyr Pro Asn Trp Arg Leu Pro Ile Ala
                725                 730                 735

Asp Gly Glu Gly His Pro Val Thr Leu Glu Glu Ile Thr Ala Ser Pro
            740                 745                 750

Arg Leu His Ala Leu Met Glu Val Leu Arg Pro Arg Lys Pro Arg Thr
        755                 760                 765

Ala Pro Pro Gly Glu Arg Arg Pro
    770                 775

## Description

### [Technical Field]

The present invention relates to a method for producing a starch-containing food and the like. More particularly, it relates to a method for producing a starch-containing food, including reacting amylomaltase derived from the genus Corynebacterium or the genus Streptomyces, and the like.

### [Background Art]

There are various problems in the field of food industry. For example, consumers' tastes are becoming more diverse and sophisticated, along with which the development of foods with new mouthfeel and flavor that have never existed before is desired. Since the quality of many foods is degraded over time, construction of means to reduce such time-related degradation of quality is also one of the major problems. Furthermore, since the amount of raw materials for food products is limited, establishment of a manufacturing method with minimal loss in the manufacturing process is also recognized as an important problem.

As one option for solving the above-mentioned problems in the field of food industry, a means characterized by modifying the properties of food with an enzyme has been reported. For example, patent document 1 discloses a method for modifying a food by utilizing β-amylase. In addition, patent document 2 discloses a method for producing cooked rice food or processed wheat food by utilizing transglucosidase. Furthermore, patent document 3 discloses a method for producing an enzyme-treated starch grain that is resistant to aging, by utilizing 4-α-glucanotransferase.

Amylomaltase is known as one of the enzymes that can be used for producing foods. Amylomaltase (hereinafter sometimes to be referred to as "AM" in the present specification) is an enzyme that catalyzes the decomposition of amylose in starch and the elongation of amylopectin sugar chains, and is known to exist widely in nature. The organisms that produce amylomaltase include microorganisms such as Escherichia coli. For food processing, heat-resistant amylomaltase derived from the genus Thermus bacterium such as Thermus flavus, Thermus aquaticus and Thermus thermophilus (hereinafter sometimes to be referred to as "Tt" in the present specification) is generally used (patent document 3, patent document 4, non-patent document 1).

Corynebacterium glutamicum (hereinafter sometimes referred to as "Cg" or "Coryne" in the present specification), which is classified in the genus Corynebacterium, is also known as a microorganism that produce amylomaltase. Corynebacterium glutamicum is a microorganism isolated in Japan in 1957 as a microorganism that excretes L-glutamic acid into the medium. It is a non-motile, aerobic gram-positive bacterium belonging to the mycolic acid-containing actinomycete group and has no sporulation ability. At present, over 2 million tons of sodium L-glutamate (Umami component) is produced worldwide by a fermentation method using the bacteria. Corynebacterium glutamicum is used for producing many useful substances such as amino acids such as lysine, nucleic acids, and organic acids, in addition to glutamic acid. However, there is no finding as to the effect of amylomaltase derived from Corynebacterium glutamicum on edible starch or starch in food products. It is known that amylomaltase derived from the genus Streptomyces bacterium (e.g., Streptomyces avermitilis, Streptomyces cinnamoneus, Streptomyces griseus, Streptomyces thermoviolaceus, Streptomyces violaceoruber and the like) can be used as a food additive. Amylomaltase derived from Streptomyces is known to show a comparatively high amino acid identity (about 40%) with amylomaltase derived from Corynebacterium glutamicum, and is considered to have enzyme property similar to that of amylomaltase derived from Corynebacterium glutamicum. However, there is also no finding as to the effect of amylomaltase derived from the genus Streptomyces on edible starch or starch in food products.

### [Document List]

### [Patent documents]

patent document 1: JP-B- 5715346
patent document 2: JP-B- 4475276
patent document 3: JP-B- 5944839
patent document 4: JP-B- 4187305

### [non-patent document]

non-patent document 1: Nguyen DH. et al., (2014) Modification of rice grain starch for lump-free cooked rice using thermostable disproportionating enzymes. Food Research International 63: 55-61.

### [Summary of Invention]

### [Technical Problem]

When a heat-resistant enzyme is used in the manufacturing process for the purpose of modifying food, the temperature characteristic of the heat-resistant enzyme may pose a problem. For example, heat-resistant enzyme retains its activity even after the starch in the food is gelatinized, due to which it also modifies the gelatinized starch, possibly causing problems of scorching and the like (it is difficult to have the enzyme act only on ungelatinized starch). In addition, many heat-resistant enzymes have low activity in the temperature range (refrigerating to room temperature) often used during storage, mixing, and forming processes in food manufacturing processes. When they are allowed to act in these processes, the effect becomes low, and addition of a large amount of the enzyme becomes necessary. Furthermore, when the heat resistance of the enzyme is high, a high temperature, long-term step is required to deactivate the enzyme in the food manufacturing process, and an influence on the food ingredients is expected to be high. Depending on the kind of food, moreover, heating strength may be insufficient and the enzyme may not be deactivated.

### [Solution to Problem]

The present inventors have conducted intensive studies of the above-mentioned problems and found that (1) CgAM acts only on ungelatinized starch because the enzyme is deactivated before the starch in the food is heat gelatinized, (2) CgAM has higher activity than TtAM in the temperature range (regrigelating to room temperature) that is often used in food manufacturing processes, and (3) CgAM is easily deactivated by heating, and the like. In addition to these, they have surprisingly found effects completely different from those of TtAM, for example, that (4) CgAM can suppress hydrogenation due to aging while maintaining the original physical properties of starch, (5) the rice added with CgAM shows improved stickiness and, while maintaining the stickiness, time-related degradation of mouthfeel can be suppressed, (6) the rice cooked with CgAM remarkably reduces the occurrence of scorching during cooking, and (7) the A blood glucose level AUC of rats that ate CgAM-modified sucrose or starch as measured 2 hr after eating is lower than that of rats that ate TtAM-modified sucrose or starch as measured 2 hr after eating. Based on such findings, they have conducted further studies and completed the present invention.

That is, the present invention provides the following.
[1] A method for producing a starch-containing food, comprising reacting an actinomycete-derived amylomaltase with starch in a raw material.
[2] The production method of [1], wherein the actinomycete is the genus Corynebacterium or the genus Streptomyces.
[3] The production method of [1] or [2], wherein the actinomycete is selected from the group consisting of Corynebacterium glutamicum, Streptomyces avermitilis, Streptomyces cinnamoneus, Streptomyces griseus, Streptomyces thermoviolaceus, and Streptomyces violaceoruber.
[4] The production method of any of [1] to [3], wherein the starch-containing food is selected from the group consisting of a rice processing food, a wheat processing food, a potato processing food, a corn processing food, a tapioca processing food, and a processing food containing one or more kinds of starch extracted from rice, wheat, potato, corn, or tapioca.
[5] The production method of any of [1] to [4], wherein the starch-containing food comprises sucrose.
[6] A method for producing a starch-containing food product, comprising reacting an actinomycete-derived amylomaltase with starch in a raw material, wherein the amylomaltase has the amino acid sequence shown in SEQ ID NO: 1, 3, 4, 5, or 6, or an amino acid sequence not less than 90% identical to the amino acid sequence.
[7] A method for modifying property of a starch-containing food, comprising reacting an actinomycete-derived amylomaltase with starch in a raw material.
[8] The method of [7], wherein the actinomycete is the genus Corynebacterium or the genus Streptomyces.
[9] The method of [7] or [8], wherein the actinomycete is selected from the group consisting of Corynebacterium glutamicum, Streptomyces avermitilis, Streptomyces cinnamoneus, Streptomyces griseus, Streptomyces thermoviolaceus, and Streptomyces violaceoruber.
[10] The production method of any of [7] to [9], wherein the starch-containing food is selected from the group consisting of a rice processing food, a wheat processing food, a potato processing food, a corn processing food, a tapioca processing food, and a processing food containing one or more kinds of starch extracted from rice, wheat, potato, corn, or tapioca.
[11] The production method of any of [7] to [10], wherein the starch-containing food comprises sucrose.
[12] A method for modifying property of a starch-containing food product, comprising reacting an actinomycete-derived amylomaltase with starch in a raw material, wherein the amylomaltase has the amino acid sequence shown in SEQ ID NO: 1, 3, 4, 5, or 6, or an amino acid sequence not less than 90% identical to the amino acid sequence.
[13] An agent for modifying property of a starch-containing food, comprising an actinomycete-derived amylomaltase.
[14] The agent of [13], wherein the actinomycete is the genus Corynebacterium or the genus Streptomyces.
[15] The agent of [13] or [14], wherein the actinomycete is selected from the group consisting of Corynebacterium glutamicum, Streptomyces avermitilis, Streptomyces cinnamoneus, Streptomyces griseus, Streptomyces thermoviolaceus, and Streptomyces violaceoruber.
[16] The agent of any of [13] to [15], wherein the starch-containing food is selected from the group consisting of a rice processing food, a wheat processing food, a potato processing food, a corn processing food, a tapioca processing food, and a processing food containing one or more kinds of starch extracted from rice, wheat, potato, corn, or tapioca.
[17] The agent of any of [13] to [16], wherein the starch-containing food comprises sucrose.
[18] An agent for modifying property of a starch-containing food, comprising an amylomaltase having the amino acid sequence shown in SEQ ID NO: 1, 3, 4, 5, or 6, or an amino acid sequence not less than 90% identical to the amino acid sequence.

In one embodiment of the present invention, the present invention provides the following.
[1A] A method for producing a starch-containing food, comprising reacting an amylomaltase derived from the genus Corynebacterium or the genus Streptomyces with starch in a raw material.
[2A] The production method of [1A], wherein the starch-containing food is selected from the group consisting of a rice processing food, a wheat processing food, a potato processing food, a corn processing food, a tapioca processing food, and a processing food containing one or more kinds of purified starch extracted from rice, wheat, potato, corn, or tapioca.
[3A] The production method of [1A] or [2A], wherein the amylomaltase has the amino acid sequence shown in SEQ ID NO: 1, 3, 4, 5, or 6, or an amino acid sequence not less than 90% identical to the amino acid sequence.
[4A] A method for modifying property of a starch-containing food, comprising reacting an amylomaltase derived from the genus Corynebacterium or the genus Streptomyces with starch in a raw material.
[5A] The production method of [4A], wherein the starch-containing food is selected from the group consisting of a rice processing food, a wheat processing food, a potato processing food, a corn processing food, a tapioca processing food, and a processing food containing one or more kinds of purified starch extracted from rice, wheat, potato, corn, or tapioca.
[6A] The production method of [4A] or [5A], wherein the amylomaltase has the amino acid sequence shown in SEQ ID NO: 1, 3, 4, 5, or 6, or an amino acid sequence not less than 90% identical to the amino acid sequence.
[7A] An agent for modifying property of a starch-containing food, comprising an amylomaltase derived from the genus Corynebacterium or the genus Streptomyces.
[8A] The agent of [7A], wherein the starch-containing food is selected from the group consisting of a rice processing food, a wheat processing food, a potato processing food, a corn processing food, a tapioca processing food, and a processing food containing one or more kinds of purified starch extracted from rice, wheat, potato, corn, or tapioca.
[9A] The agent of [7A] or [8A], wherein the amylomaltase has the amino acid sequence shown in SEQ ID NO: 1, 3, 4, 5, or 6, or an amino acid sequence not less than 90% identical to the amino acid sequence.

### [Advantageous Effects of Invention]

According to the present invention, a starch-containing food that has a preferable mouthfeel different in quality from existing sugar-modifying enzymes, is less susceptible to time-related degradation, and is less likely to raise blood glucose level after eating can be produced, and a starch-containing food provided with the above-mentioned properties and free from a problem specific to existing heat-resistant amylomaltase (e.g., scorching on cookware) in some cooking styles can be produced.

### [Brief Description of Drawings]

Fig. 1 shows time-course changes in the gel intensity of a 10% starch gel when amylomaltase derived from each microorganism was added.
Fig. 2 shows the amount of scorched part in cooked rice when amylomaltase derived from each microorganism was added.
Fig. 3 is a graph showing that the Corynebacterium glutamicum-derived amylomaltase and the amylomaltase derived from the genus Streptomyces have similar effects in modifying the property of a starch gel.
Fig. 4 is a graph showing that the Corynebacterium glutamicum-derived amylomaltase and the amylomaltase derived from the genus Streptomyces have similar actions in amylose decomposition.
Fig. 5 shows difference in the sugar chain transferring action between Thermus thermophilus-derived amylomaltase and Corynebacterium glutamicum-derived amylomaltase.
Fig. 6 shows the schedule of blood glucose level measurement test using rats.
Fig. 7 shows that rats after eating dextrin and sucrose modified using a Corynebacterium glutamicum-derived amylomaltase showed a suppressed increase in the blood glucose level by eating the components (upper Figure: Δblood glucose level rise curve, lower Figure: Δblood glucose level AUC).
Fig. 8 shows that rats after eating α non-glutinous rice starch modified using a Corynebacterium glutamicum-derived amylomaltase showed a suppressed increase in the blood glucose level by eating the component (upper Figure: Δblood glucose level rise curve, lower Figure: Δblood glucose level AUC).
Fig. 9 shows that rats after eating α non-glutinous rice starch modified using a Thermus thermophilus-derived amylomaltase did not show a suppressed increase in the blood glucose level by eating the component (upper Figure: Δblood glucose level rise curve, lower Figure: Δblood glucose level AUC) .
Fig. 10 shows that rats after eating rice cooked with addition of a Corynebacterium glutamicum -derived amylomaltase showed a suppressed increase in the blood glucose level by eating the rice (upper Figure: Δblood glucose level rise curve, lower Figure: Δblood glucose level AUC).

### [Description of Embodiments]

The present invention is explained in detail in the following.

### 1. Production method of starch-containing food

The present invention provides a method for producing a starch-containing food, including reacting an actinomycete-derived amylomaltase with starch in a raw material (hereinafter sometimes to be referred to as "the production method of the present invention").

Amylomaltase (EC number: 2.4.1.25) is an enzyme that catalyzes a chemical reaction that transfers a portion of 1,4-α-glucan chain to 4-OH group of glucose or another α-glucan. When the substrate is sufficiently large, amylomaltase causes an intramolecular transfer, and the resultant product has a cyclic structure.

In the present specification, the "a-glucan" is α-1,4-glucan (polysaccharide with a chain-like structure and maltose as a constitutional disaccharide unit), or α-1,4-glucan having an α-1,6-branched structure. The "a-glucan" nonlimitatively includes amylose, amylopectin, starch and glycogen, waxy starch, high amylose starch, soluble starch, dextrin, starch hydrolyzate, enzyme-synthesized amylopectin by phosphorylase and the like.

In the present specification, the "cyclic glucan" includes cyclic α-1,4-glucan having α-1,4-glucoside bond alone, and branched type cyclic glucan having both a-1,4-glucoside bond and α-1,6-glucoside bond. The "branched type" means having at least one glucoside bond other than α-1,4-bond. Examples of the branched type cyclic glucan include an inner branched type cyclic glucan containing a branched structure having an α-1,6-bond inside the cyclic structure, and an outer branched type cyclic glucan having a non-cyclic structure part in addition to the cyclic structure.

The amylomaltase used in the present invention may be amylomaltase derived from the genus Corynebacterium or the genus Streptomyces, or a mutant thereof. In the present specification, the "amylomaltase derived from the genus Corynebacterium or the genus Streptomyces" means an amylomaltase produced by a bacterium (wild-type or variant strain) classified into the genus Corynebacterium or the genus Streptomyces, or an amylomaltase obtained by a genetic engineering method using the amylomaltase gene of a bacterium (wild-type or variant strain) classified into the genus Corynebacterium or the genus Streptomyces. Therefore, a recombinant amylomaltase protein expressed by a host transformed or transduced with the amylomaltase gene (wild-type or variant) obtained from a bacterium classified into the genus Corynebacterium or the genus Streptomyces also falls under the "amylomaltase derived from the genus Corynebacterium or the genus Streptomyces".

Examples of the origin of amylomaltase preferably used in the production method of the present invention include, but are not limited to, Corynebacterium glutamicum, Streptomyces avermitilis, Streptomyces cinnamoneus, Streptomyces griseus, Streptomyces thermoviolaceus, and Streptomyces violaceoruber. Examples of the amylomaltase derived from Corynebacterium glutamicum include amylomaltase having the amino acid sequence shown in SEQ ID NO: 1. Examples of the amylomaltase derived from Streptomyces avermitilis include amylomaltase having the amino acid sequence shown in SEQ ID NO: 3. Examples of the amylomaltase derived from Streptomyces cinnamoneus include amylomaltase having the amino acid sequence shown in SEQ ID NO: 4. Examples of the amylomaltase derived from Streptomyces griseus include amylomaltase having the amino acid sequence shown in SEQ ID NO: 5. Examples of the amylomaltase derived from Streptomyces violaceoruber include amylomaltase having the amino acid sequence shown in SEQ ID NO: 6. Examples of the variants thereof include amylomaltase having an amino acid sequence which is the amino acid sequence shown in SEQ ID NO: 1, 3, 4, 5 or 6 wherein one or more amino acids are added, deleted, inserted or substituted. Amylomaltase having one or more mutations may include any mutation or modification as long as it has the equivalent (or more) enzymological properties as a wild-type amylomaltase derived from Corynebacterium glutamicum, Streptomyces avermitilis, Streptomyces cinnamoneus, Streptomyces griseus, Streptomyces thermoviolaceus, or Streptomyces violaceoruber. Amylomaltase having one or more mutations in the amino acid sequence shown in SEQ ID NO: 1, 3, 4, 5, or 6 has an amino acid sequence has identity of generally not less than 70%, preferably not less than 80%, more preferably not less than 90%, further preferably not less than 95%, further more preferably not less than 98%, particularly preferably not less than 99%, with respect to the amino acid sequence shown in SEQ ID NO: 1, 3, 4, 5, or 6, and each also has the equivalent (or more) amylomaltase activity as the corresponding wild-type amylomaltase. In the present specification, the "identity" of an amino acid sequence or a base sequence refers to the degree of appearance of the same amino acid (base when base sequences are compared) between two sequences. The "identity" of a sequence can be easily determined by those skilled in the art by a method known per se.

The above-mentioned amylomaltase having the amino acid sequence shown in SEQ ID NO: 1, 3, 4, 5, or 6, or mutant amylomaltase having one or more mutations in the amino acid sequence shown in SEQ ID NO: 1, 3, 4, 5, or 6 can be produced by a method known per se. In one embodiment, amylomaltase can be prepared by inserting a base sequence (SEQ ID NO: 2) encoding an amylomaltase having the amino acid sequence shown in SEQ ID NO: 1 into an appropriate gene expression vector, expressing the vector in a protein overexpression system known per se such as Escherichia coli, and purifying same using an appropriate means. A mutant amylomaltase can be produced by first modifying a part of the base sequence shown in SEQ ID NO: 2 by using a genetic engineering method such as site-directed mutagenesis, inserting same into a gene expression vector, expressing the vector in a protein overexpression system known per se such as Escherichia coli, and purifying same.

In the present specification, the activity of amylomaltase is measured and defined as follows. That is, an amylomaltase solution is added to a 30 mM Tris-HCl buffer (pH 7.5) containing 0.05% potato starch and 0.05% maltose, and the mixture is reacted for a certain period of time in a water bath at 30°C - 70°C (which may vary depending on the kind of amylomaltase), and heated at 96°C for 5 min to discontinue the reaction. 0.1 ml of this reaction mixture and 1 ml of iodine solution (0.02% iodine, 0.2% potassium iodide) are mixed and the absorbance at 600 nm is measured. A value obtained by subtracting the absorbance at the time of enzyme addition from the absorbance of the blank in which milli-Q water is mixed instead of the enzyme solution was used as the activity value, and the amount of enzyme necessary for reducing the absorbance at 600 nm by 1 per minute was used as 1 unit (U).

The optimum temperature of Corynebacterium glutamicum-derived amylomaltase used in the production method of the present invention is 30°C - 38°C. The optimum temperature of amylomaltase derived from the genus Streptomyces used in the production method of the present invention is 45°C - 55°C (e.g., 50°C). In the present specification, the "optimum temperature" means the temperature at which the activity is highest when amylomaltase is allowed to act for 10 min at each temperature in 30 mM Tris-HCl buffer (pH 7.5) in the presence of 0.05% potato starch and 0.05% maltose. The optimum temperature of the amylomaltase derived the genus Thermus thermophilus is about 70°C.

The optimum pH of the Corynebacterium glutamicum-derived amylomaltase used in the production method of the present invention is 6 to 7. The optimum pH of the amylomaltase derived from the genus Streptomyces used in the production method of the present invention is 4 to 9 (e.g., 7). In the present specification, the "optimum pH" means the pH at which the activity is highest when amylomaltase is allowed to act for 10 min at 37°C in 30 mM acetate buffer (pH3 - 5.5) or 30 mM phosphate buffer (pH 6 - 7) or 30 mM Tris-HCl buffer (pH 7.5 - 10) in the presence of 0.1% potato starch and 0.05% maltose.

Regarding the heat resistance of the Corynebacterium glutamicum-derived amylomaltase used in the production method of the present invention, the enzyme is stable at not more than 40°C. As for the heat resistance of the amylomaltase derived from the genus Streptomyces used in the production method of the present invention, it is stable at not more than 50°C. In the present specification, the "heat resistance" means that amylomaltase does not lose activity in 30 mM Tris-HCl buffer (pH 7.5) for 10 min.

The pH stability of the Corynebacterium glutamicum-derived amylomaltase used in the production method of the present invention is 6 to 8. The pH stability of the amylomaltase derived from the genus Streptomyces used in the production method of the present invention is 4 to 9. In the present specification, the "pH stability" means that amylomaltase does not lose activity in a buffer (25°C) for about 18 hr.

The amount of the amylomaltase derived from the genus Corynebacterium glutamicum or amylomaltase derived from the genus Streptomyces used in the production method of the present invention is not particularly limited as long as a desired effect can be obtained. It may be generally 0.00001 - 10000U, preferably 0.0001 - 1000U, more preferably 0.001 - 100U, further preferably 0.01 - 10U, particularly preferably 0.1 - 1U, per 1 g of starch in the raw material. In one embodiment, when the starch-containing food produced by the production method of the present invention is cooked rice, generally 0.00001 - 10000U, preferably 0.0001 - 1000U, more preferably 0.001 - 100U, further preferably 0.01 - 10U, particularly preferably 0.1 - 1U, of amylomaltase can be added per 1 g of dried rice before boiling.

The timing of adding the amylomaltase derived from the genus Corynebacterium or Streptomyces is not particularly limited as long as a desired effect is obtained. The timing of adding the enzyme may be appropriately set in consideration of the kind of food to be produced and the cooking procedure thereof, the kind of starch raw material to be used, the preference of the consumer and the like, and is not particularly limited. Therefore, the enzyme may be added at any time before cooking, during cooking, after cooking, or before eating the starch-containing food. In one embodiment, it is preferable to add and mix amylomaltase to the raw material to produce or process a food, or allow the enzyme to act on the starch in the raw material by adding and mixing amylomaltase to the food under manufacturing or processing. In a specific example when producing cooked rice, dry rice is washed with water, the washed rice is mixed with water and the amylomaltase derived from the genus Corynebacterium or Streptomyces and stood at room temperature (10°C - 30°C) for a certain period of time (e.g., 0.5 - 2 hr), and thereafter cooked by a general method, whereby the cooked rice having the desired effect of the present invention can be prepared.

In the production method of the present invention, the temperature and time at which the amylomaltase derived from the genus Corynebacterium or Streptomyces is allowed to act on starch in the raw material are not particularly limited as long as a desired effect is obtained. The action temperature of the enzyme may be appropriately set in consideration of the amount of the enzyme to be used, the kind of food to be produced and the cooking procedure thereof, the kind of starch raw material to be used, the preference of the consumer and the like, and is not particularly limited. However, it should be taken into consideration that the optimal temperature of the amylomaltase derived from the genus Corynebacterium or Streptomyces is lower than that of amylomaltase derived from a heat resistant bacterium. Therefore, in one embodiment, the temperature of food at the time of addition of the enzyme may be generally 1 - 100°C, preferably 5 - 50°C, more preferably 10 - 45°C, further preferably 20 - 40°C, particularly preferably 30 - 37°C. While the action time of the enzyme is not particularly limited, it may be generally 0.1 - 48 hr, preferably 0.2 - 36 hr, more preferably 0.5 - 24 hr, further preferably 0.8 - 20 hr, particularly preferably 1 - 18 hr. For example, when cooked rice is produced with addition of 0.1 - 1U amylomaltase per 1 g of dried rice, the washed rice is mixed with water and the amylomaltase derived from the genus Corynebacterium or Streptomyces, and modified with the enzyme for 0.1 - 18 hr at 4 - 40°C, preferably 0.25 - 6 hr at 10 - 38°C, more preferably 0.5 - 1 hr at 20 - 37°C, and thereafter cooked by a general method, whereby the cooked rice having the desired effect of the present invention can be prepared.

In the production method of the present invention, the pH environment when the amylomaltase derived from the genus Corynebacterium or the genus Streptomyces is acted on starch in the raw material is adjusted in advance to, for example, pH 6 - pH 7 as described above in consideration of the optimum pH and pH stability of the enzyme by using, where necessary, a pH adjustor that can be added to food.

The starch-containing food produced by the production method of the present invention includes any food containing starch. The origin of the starch contained in the starch-containing food is not particularly limited, and may be one or more selected from the group consisting of rice starch, wheat starch (including wheat, barley, rye and the like), potato starch, ocarina starch, corn starch, soybean starch, and tapioca starch. Specific examples of the starch-containing food include, but are not limited to, rice processing food, wheat processing food, potato processing food, corn processing food, tapioca processing food, and a processing food containing one or more kinds of starch extracted from rice, wheat, potato, corn, or tapioca. In the present specification, examples of the rice processing food include, but are not limited to, cooked rice and processed product thereof (festive red rice, pilaf, cooked rice with fish and vegetables mixed in advance (*takikomigohan*), rice porridge, risotto, rice ball, sushi, rice cake, rice-cake sweets etc.), rice noodle and processed product thereof and the like. Examples of the wheat processing food include, but are not limited to, noodles such as pasta, ramen, Japanese wheat noodle and the like, bread, bread dough such as piazza, naan and the like, confectionery such as cookie, cake and the like, and the like. Examples of the potato processing food include, but are not limited to, potato salad, fried potato, boiled potato, mashed potato, potato snacks such as potato chips and the like, and the like. Examples of the corn processing food include, but are not limited to, taco, tortilla, arepa, doughs thereof and the like. Examples of the tapioca processing food include, but are not limited to, steamed dumpling containing tapioca, purine containing tapioca, and the like. Examples of the processing food containing one or more kinds of purified starch extracted from rice, wheat, potato, corn, or tapioca include, but are not limited to, boiled fish paste, crab stick, sausage, hamburg and the like.

### 2. Method for modifying property of starch-containing food

The present invention also provides a method for modifying property of a starch-containing food, including reacting an actinomycete-derived amylomaltase with starch in a raw material (hereinafter sometimes to be referred to as "the method of the present invention").

The amylomaltase of actinomycete to be used in the method of the present invention, the amount thereof to be added, the timing of addition, the reaction temperature, the reaction pH, the preparation method, etc., and the kind of the starch-containing food are all the same as those explained in "1. Production method of starch-containing food".

### 3. Agent for modifying property of starch-containing food

The present invention also provides an agent for modifying property of a starch-containing food product, including an actinomycete-derived amylomaltase (hereinafter sometimes to be referred to as "the agent of the present invention").

The amount of the actinomycete-derived amylomaltase (e.g., amylomaltase derived from the genus Corynebacterium or Streptomyces) contained in the agent of the present invention is not particularly limited. It may be any as long as the amylomaltase can be added at a proportion of generally 0.00001 - 10000U, preferably 0.0001 - 1000U, more preferably 0.001 - 100U, further preferably 0.01 - 10U, particularly preferably 0.1 - 1U, per 1 g of starch in the raw material.

The agent of the present invention may contain components other than the amylomaltase derived from the genus Corynebacterium or Streptomyces. Examples of such component include, but are not limited to, excipient, buffering agent, suspension, stabilizer, preservative, antiseptic and seasoning and the like.

The dosage form of the agent of the present invention is not particularly limited, and may be a solid form such as powder form, granular form or the like, a liquid form, or a paste form.

Other aspects of the agent of the present invention such as use conditions and the like can be appropriately set by those of ordinary skill in the art by reference to the conditions explained in "1. Production method of starch-containing food product".

While the present invention is explained in more detail in the following Examples, the present invention is not limited in any manner by the Examples.

### [Example]

### [Example 1] Effect on property of starch gel

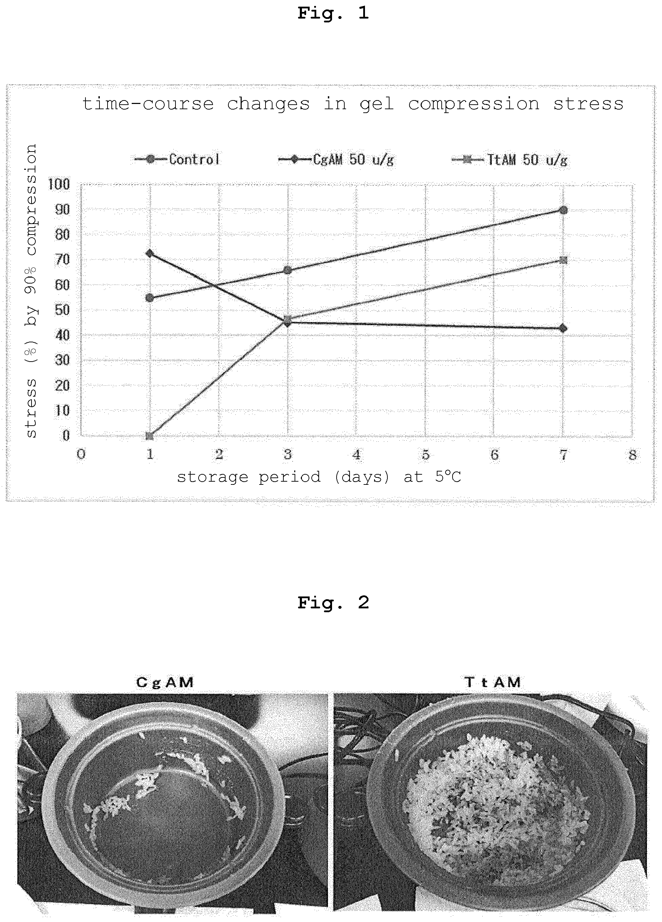

To a 10% suspension of rice starch was added Corynebacterium glutamicum-derived amylomaltase or Thermus thermophilus-derived amylomaltase (hereinafter sometimes referred to as "CgAM" or "TtAM" and the like) at 50 U/g starch, and the mixture was reacted at 37°C for 1 hr while stirring. The mixture was heated at 98°C for 10 min, and cooled to 50°C. The starch gel after cooling was molded into a cylindrical shape having a diameter of 5 mm and a height of 20 mm by using a plastic cylindrical tube and gelled by storing at 5°C for 1 - 7 days. The obtained starch gel was taken out of the tube and formed into a cylindrical shape having a diameter of 5 mm and a height of 5 mm by using a razor blade, and then subjected to a compression test using a texture analyzer (TA-XT Plus). The gel strength was measured on day 1, day 3 and day 7. The results are shown in Fig. 1.

As shown in Fig. 1, when amylomaltase was not added (Control), the starch gel gradually hardened during storage in a refrigerator along with the aging of the starch. When TtAM was added, the viscosity of starch was markedly lowered, and it took time to form a gel. It hardened rapidly after gelation. When CgAM was added, a gel having properties similar to those of the starch gel without addition of enzyme was formed. Furthermore, the gel scarcely hardened even after several days.

### [Example 2] Improvement of mouthfeel of cooked rice

Dried rice (150 g) was washed, water and amylomaltase derived from each microorganism (1.0 U/g dried rice) were added to adjust to 325 g, and the mixture was stood at room temperature (20°C) for 1 hr. This was cooked with a rice cooker SR-13GP (Panasonic). The obtained cooked rice was taken out of the rice cooker, transferred to a container with a plastic cover, and stood at room temperature for 1 hr. Then, sensory evaluation was performed by four professional panels.

The sensory evaluation was performed for three items of "softness", "pebbly feel", and "stickiness". In the present specification, the "softness" means that the resistance felt when masticating cooked rice is small, "pebbly feel" means that the grains remain strong during masticating, and "stickiness" means that the rice grains are attached to each other. The evaluation criteria were as follows. With the "softness", "pebbly feel", and "stickiness" of cooked rice prepared without adding enzymes as a standard (0 point), the degree of strength in each item was evaluated from -2 to +2 points (in increments of 0.5 point). Samples other than the standard product (enzyme-free) were presented in blind. The score was an average of the scores of the four professional panels. The results are shown in Table 1.

**[Table 1]**

| | enzyme-free | TtAM 1.0u/g | CgAM 1.0u/g |
|---|---|---|---|
| softness | 0 (standard) | -0.63 | 0.50 |
| pebbly feel | 0 (standard) | 1.00 | 0.50 |
| stickiness | 0 (standard) | -0.75 | 0.75 |

As shown in Table 1, different from the cooked rice prepared by adding TtAM, the cooked rice prepared by adding CgAM changed into a soft mouthfeel with high stickiness.

### [Example 3] Suppression of time-related degradation of cooked rice

Cooked rice was prepared under the same conditions as in Example 2. This rice was left standing for 18 hr in a thermostatic tank set to 20°C and then subjected to sensory evaluation. The conditions and method for sensory evaluation were the same as those used in Example 2. The results are shown in Table 2.

**[Table 2]**

| <the day of cooking rice> | | | |
|---|---|---|---|
| | enzyme-free | TtAM 1.0u/g | CgAM 1.0u/g |
| softness | 0 (standard) | -0.63 | 0.50 |
| pebbly feel | 0 (standard) | 1.00 | 0.50 |
| stickiness | 0 (standard) | -0.75 | 0.75 |

| <after 20°C, 18 hr> | | | |
|---|---|---|---|
| | enzyme-free | TtAM 1.0u/g | CgAM 1.0u/g |
| softness | -0.75 | -1.00 | 0.13 |
| pebbly feel | -0.13 | 1.25 | 0.63 |
| stickiness | -1.13 | -1.00 | -0.25 |

As shown in Table 2, the softness of cooked rice without addition of enzyme decreased by preservation at 20°C for 18 hr and the stickiness thereof became low. When TtAM was added, the softness and stickiness were low immediately after cooking rice, and changes in the properties were small after preservation at 20°C for 18 hr. On the other hand, when CgAM was added, softness and stickiness increased immediately after cooking rice, and the softness and stickiness decreased somewhat after preservation at 20°C for 18 hr. Compared with TtAM, however, the properties were generally maintained close to those of cooked rice prepared without addition of enzyme on the day of cooking.

### [Example 4] Suppression of scorching

Cooked rice was prepared under the same conditions as in Example 2, and the amount of scorching was examined. Note that the amount of scorching refers to the weight of the portion of rice that remained sticking to the inner pot when the inner pot containing the cooked rice was taken out of the rice cooker immediately after completion of cooking rice and inverted to drop the cooked rice. The results are shown in Table 3 and Fig. 2. The values shown in Table 3 are average values calculated from the values obtained in three tests.

**[Table 3]**

| | enzyme-free | TtAM 1.0u/g | CgAM 1.0u/g |
|---|---|---|---|
| amount of scorch (g) | 17.2 | 52.20 | 15.30 |

As shown in Table 3 and Fig. 2, when TtAM was added, candy-like scorch occurred on the bottom of the pot; however, almost no scorch occurred with CgAM.

### [Example 5] suppression of time-related degradation of wheat starch-containing food

To confirm a time-related degradation-suppressive effect of CgAM on a wheat starch-containing food, plain bread blended with CgAM was prepared, and the degree of degradation of the mouthfeel of the plain bread after preservation for a given period after the preparation was verified. To be specific, water was weighed in a metal container attached to a breadmaker (MK SEIKO CO., LTD., HBK-100), and a predetermined amount of CgAM (test section 1: no addition, test section 2: 0.01 U/g wheat flour, test section 3: 0.1 U/g wheat flour, test section 4: 1.0 U/g wheat flour) was added. Then a pre-mixed mixture of hard flour, sugar, skim milk, and salt was added thereto. Water and then dry yeast and shortening were added, and bread was prepared by kneading and baking according to the predetermined program (plain bread, baking color: normal). The actual amount of each component is shown in the following Table 4. After completion of baking, the bread was cooled by allowing to stand at room temperature for 1.5 hr, then sliced to a thickness of 2 cm, enclosed in a vinyl bag with a chuck, and stored at a temperature of 10°C and a humidity of 50% for 2 days.

**[Table 4]**

| basic formulation | | |
|---|---|---|
| | formulation | 1 test section weight (g) |
| hard flour | 100.00% | 280.0 |
| sugar | 8.0% | 22.4 |
| skim milk | 2.00% | 5.6 |
| sodium chloride | 1.50% | 4.2 |
| dry yeast | 1.1% | 3.0 |
| shortening | 5% | 14.0 |
| water | 66% | 184.8 |
| total amount | | 514.0 |

| | | |
|---|---|---|
| "%" is baker's %. | | |

The mouthfeel ("drying") of the plain bread stored under the aforementioned conditions was evaluated by sensory evaluation by three professional panels. The evaluation criteria used in the sensory evaluation were as follows.
×: strongly dried
Δ: dried
○: a little dried
⊙: moist

The results are shown in the following Table 5.

**[Table 5]**

| | test section 1 | test section 2 | test section 3 | test section 4 |
|---|---|---|---|---|
| enzyme (amount added) | no addition | CgAM 0.01 U/g wheat flour | CgAM 0.1 U/g wheat flour | CgAM 1 U/g wheat flour |
| sensory evaluation | × | Δ | ○ | ⊙ |

As shown in Table 5, in the bread containing CgAM, the dryness of the plain bread was suppressed even after 2 days from baking.

### [Example 6] Effect on property of potato starch

Potato flakes ("potato flakes", Taimou Kabushiki Kaisha) were weighed (40 g in one test section) and mixed with 120 g of water. CgAM was added thereto and the mixture was stirred with a spatula until uniformity. The obtained mixture was stood at room temperature for 30 min. After standing for 30 min, the mixture was divided into 3 packs by 50 g each and tightly sealed with a vacuum sealer. The tightly sealed packs were heated in boiling water for 30 min, and then cooled with running water to about 25°C. The pack was opened and the content was dispensed into a 24 well microplate. This was stored in a refrigerator (5°C) and subjected to a compression test using a texture analyzer on the day of dispensing, one day later, one week later, and two weeks later and the hardness of the paste was measured.

The amount of CgAM used in this Example was 0.1 U or 1.0 U per 1 g of the dried potato flakes.

The measurement conditions of the texture analyzer used for the property measurement are as follows. apparatus: texture analyzer ("TA-XT Plus" (EKO INSTRUMENTS CO., LTD.)) diameter 5 mm stainless spherical plunger measurement conditions: compression rate 1 mm/sec, the center of the model potato salad filled in a plate was compressed (penetrated) 50% and the maximum stress was recorded (N=3).

The results are shown in the following Table 6 (compression stress).

**[Table 6]**

| | | storage days at 5°C | | | |
|---|---|---|---|---|---|
| | | 0 | 1 | 7 | 14 |
| potato starch | Control | 26.4 | 108.3 | 281.4 | 325.9 |
| | CgAM 0.1 U/g potato flakes | 36.6 | 103.0 | 220.4 | 221.1 |
| | CgAM 1.0 U/g potato flakes | 23.9 | 99.2 | 181.5 | 184.1 |

The numerical value is maximum stress (mean) by 50% compression, and the unit is g.

As shown in Table 6, CgAM was shown to suppress time-course hardening of the model potato salad.

### [Example 7] Starch property modification 1 of amylomaltase derived from the genus Streptomyces

Amylomaltase derived from the genus Streptomyces and Corynebacterium glutamicum-derived amylomaltase are known to have relatively high amino acid identity and their functions are expected to be similar. The following experiment was conducted to demonstrate same.

To 10 mM phosphate buffer (pH 7) was added rice starch (SIGMA) to give a 10% suspension, amylomaltase derived from CgAM, TtAM or Streptomyces avermitilis (hereinafter sometimes to be referred to as "SaAM") was further added at 50 U/g starch, and the mixture was reacted at 37°C for 1 hr while stirring. The mixture was heated at 98°C for 10 min to deactivate the enzyme, and cooled to 50°C. The starch gel after cooling was dispensed to a cylindrical tube having a diameter of 5 mm and gelled by storing at 5°C for 1 - 7 days. The obtained starch gel was taken out of a refrigerator at the time points of one day, 3 days and 7 days later with the preparation day of the starch paste as day 0, and formed into a cylindrical shape having a diameter of 5 mm and a height of 5 mm by using a razor blade. It was placed on the stage of a texture analyzer (TA-XT Plus) such that the cut surface is up and down, and subjected to a compression test in which the gel strength was measured.

The texture analyzer used for the property measurement and the measurement conditions are as follows.
apparatus: texture analyzer ("TA-XT Plus" (EKO INSTRUMENTS CO., LTD.)) diameter 15 mm acrylic cylindrical plunger, stainless stage
measurement conditions: compression rate 0.5 mm/sec, the maximum stress (g) obtained when starch gel piece was compressed 90% was recorded (N=6 - 9).

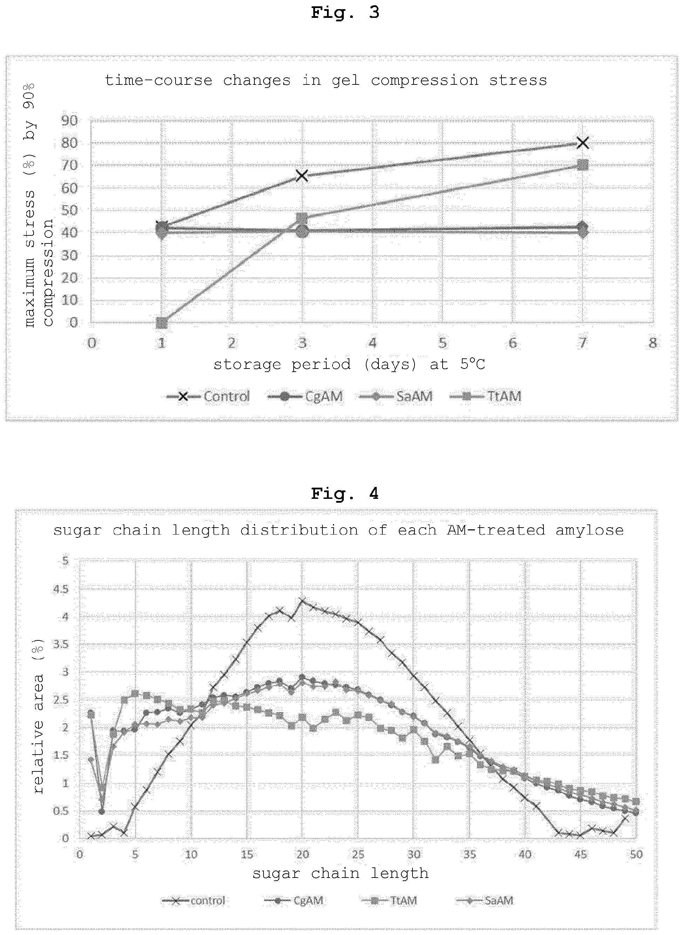

The results are shown in Fig. 3. As shown in Fig. 3, the properties of the starch gel treated with SaAM and changes thereof with time were almost the same as those of the starch gel treated with CgAM. That is, it was shown that amylomaltase derived from the genus Streptomyces and CgAM have high possibility of affording similar effects in starch modification.

### [Example 8] Starch property modification 2 of amylomaltase derived from the genus Streptomyces

Amylose (BAR-5K-1, GLICO NUTRITION CO., LTD.) was added to dimethyl sulfoxide to give a 10% solution, and the solution was diluted with milli-Q water to prepare a 1% amylose solution. To the prepared 1% amylose solution (500 µl) was added 50 µL of each amylomaltase solution (SaAM, CgAM, or TtAM) adjusted to 1 U/mL. The mixture was reacted at SaAM: 50°C, CgAM: 37°C, TtAM: 70°C for 60 min. Then, each reaction mixture was heated at 100°C for 10 min to deactivate the enzyme and cooled to room temperature. After cooling, each reaction mixture was diluted with milli-Q water such that the amylose concentration was 0.1%, and the distribution of sugar chain length of the amylose degradant contained in the diluted reaction mixture was analyzed using ion chromatography (Dionex). The relative value of the peak area of each sugar chain to the total peak area was calculated. The results are shown in Fig. 4.

As shown in Fig. 4, the sugar chain length distribution obtained by treating amylose with CgAM and the sugar chain length distribution obtained by treating amylose with SaAM were extremely similar. Also in this result, it was shown that the starch-modifying property of amylomaltase derived from the genus Streptomyces was similar to that of CgAM.

### [Example 9] Study of sugar chain transferring function of CgAM and TtAM

Each rice starch (1.5g) was added to 28.5 ml of 50 mM phosphate buffer (pH 6.0) to prepare a 5% starch suspension. The obtained starch suspension was placed in a standing pouch, and the starch was gelatinized by heating at 100°C for 15 min (gelatinized starch solution 1). In addition, 1.5 g of glucose (sometimes referred to as "G1") or sucrose (sometimes referred to as "suc") was added to 28.5 ml of 50 mM phosphate buffer (pH 6.0) to prepare a 5% G1 solution or suc solution and the solution was dispensed into a 1.5 mL tube. Then, gelatinized starch solution 1 (500 µL) and G1 solution or Suc solution or milli-Q water (500 µL) were mixed to give a mixed solution (1 mL) .

Next, 2.5 U/mL CgAM solution and 2.5 U/mL TtAM solution were prepared. The obtained enzyme solutions (100 µL) were each added (enzyme 100 U per 1 g of starch) to the mixed solution (1 mL) adjusted as mentioned above. After addition of the enzyme solution, the reaction mixture added with CgAM was heated to 30°C, and the reaction mixture added with TtAM was heated to 50°C. The control added with milli-Q water instead of the enzyme solution was heated to 30°C. After 24 hr, each enzyme in the reaction mixture was deactivated by heating at 100°C for 10 min. Each reaction mixture was subjected to TLC analysis later.

TLC analysis was performed as follows. As a standard, 2 µL of three kinds of 0.5% sugar solutions were spotted. The three kinds of sugar solutions used as the standards were as follows.
sugar solution 1: glucose (G1), maltose (G2), maltotriose (G3), maltotetraose (G4), maltopentaose (G5), maltohexaose (G6) and maltoheptaose (G7) mixture
sugar solution 2: sucrose solution (suc)
sugar solution 3: α cyclodextrin (αCD) and β cyclodextrin (βCD) mixture

The reaction mixture after the enzyme deactivation was 5-fold diluted with milli-Q water to adjust rice starch, G1, or Suc to 0.5%, and then 2 µL was spotted.

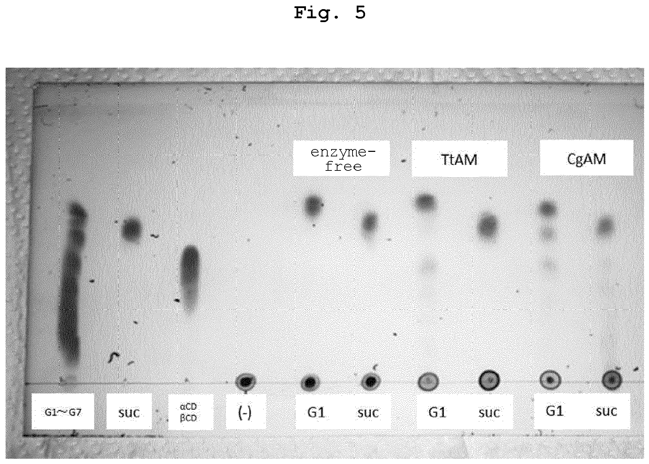

The reaction mixture was developed once, and the composition of the solvent for development was n-butanol:pyridine:milli-Q water (MQ)=6:4:1. For detection, color was developed by spraying 20% sulfuric acid/EtOH on the carrier and then heating same at 110°C for about 10 min. The results are shown in Fig. 5. In Fig. 5, "(-)" shows the lane on which a sample obtained by mixing gelatinized starch solution 1 (500 µL) and 50 mM phosphate buffer (pH 6.0) (500 µL), adding milli-Q water instead of the enzyme solution, and heating the mixture at 30°C for 24 hr was spotted.

As shown in Fig. 5, TtAM transferred a sugar chain to glucose (G1) and produced an oligosaccharide, but did not transfer a sugar chain to sucrose (suc). On the other hand, CgAM transferred a sugar chain to both glucose (G1) and sucrose (suc), and it was shown that the reaction property with sucrose was different between the both AMs.

### [Example 10] Property modification of sucrose-containing dextrin by CgAM

### (preparation of test substance)

Dextrin (Matsutani Chemical Industry Co., Ltd. Paindex #100) was dissolved with milli-Q water to give a 5% solution. Sucrose (JUNSEI CHEMICAL CO., LTD. reagent special grade) was similarly dissolved with milli-Q water to give a 5% solution. These were mixed at a 1:1 quantitative ratio, and the CgAM solution was added at 100 U per 1 g of dextrin. The same amount of milli-Q water was added to the control. The mixture was stood in a water bath at 30°C for 24 hr to perform an enzyme reaction. Thereafter, the enzyme was deactivated by heating in a hot-water bath at 100°C for 10 min. The dextrin-sucrose solution after the enzyme reaction was stored in a freezer at -80°C.

### (Animal test)

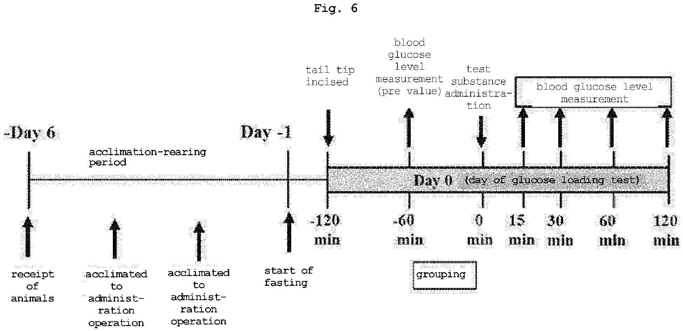

By the method described below, the blood glucose level after administration of starch was measured using rats and a suppressive effect on an increase in the blood glucose level was evaluated. The test substance was thawed with running water on the day of the blood glucose measurement test and used for the test. According to the following measurement method of the blood glucose level and the test schedule of Fig. 6, the blood glucose level was measured during fasting, 15 min, 30 min, 60 min, and 120 min after administration. The test substance was administered orally at a total amount of sugar in the test substance of 1 g/20 mL/kg. The total sugar mass analysis of the test substance was outsourced to the Japan Food Research Laboratories and measured using the phenol-sulfuric acid method.

### (Measurement method of blood glucose level)

Various glucose loading tests on rats have been performed. In this test, the glucose loading test disclosed in JP-A-2005-328776 was modified and performed.

### [Animal]

animal species and lineage: rat, Slc:Wistar (SPF)
manufacturer: Japan SLC, Inc.
sex: male
age at arrival: 6 weeks of age
quarantine, acclimation: Animals are acclimatized from arrival to grouping. Quarantine is conducted for up to day 7 with the arrival date as day 0. General condition is observed every day.

### [Rearing environment]

temperature: 22±3°C
humidity: 50±20%
lighting time: 12 hr/day

### [Feed]

kind: Labo MR stock solid feed (Nosan Corporation) or CRF-1 (Oriental Yeast Co., Ltd.)
feeding method: freely given except during the fasting period.

### [Drinking water]

kind: tap water
water feed method: freely given throughout the test period.

### [Selection and grouping of animal]

The animals to be used for the test are selected from the animals that did not show any abnormalities in the observation of general conditions during the quarantine/acclimation period. The animals are used at 7 weeks of age. The body weight is measured on the final day of quarantine/acclimation, and animals are assigned to 6 to 10 animals/group by the stratified randomization method and using the obtained body weight as an index.

### [Fasting treatment]

Fasting is started from the evening of one day before the glucose loading test and performed overnight.

### [Measurement of blood glucose level]

The vein at the tip of the tail is incised using a scalpel blade under unanesthesia. The test (blood glucose level) is performed using blood leaking from the incision surface. The measurement is performed using a self-checking glucometer "ACCU-CHEK" (Roche DC) or "glutest Neo" (Sanwa Kagaku Kenkyusho), and the blood glucose level displayed on the measuring instrument is recorded. This is used as the fasting blood glucose level. The same measuring instrument was used for the tests on the same day.

The calculation of the evaluation items was performed as follows.

Fasting blood glucose level was used as the blood glucose level at 0 minute.

Value obtained by subtracting the blood glucose level at 0 minute from the blood glucose level at each measurement time was used as "Δblood glucose level (mg/dL)".

The highest value of the Δ blood glucose level at each measurement time was used as "ΔCmax (mg/dL)" of each individual.

The value obtained by calculating the area under the Δ blood glucose level increase curve was used as "Δ blood glucose level AUC (mg/dL·min)". The calculation method was based on the method of Japanese Association for the Study of Glycemic Index.

The suppressive effect on the elevation of blood glucose level was evaluated from the value of Δ blood glucose level AUC of the test substance administration group based on the Δ blood glucose level AUC of the control group as 100.

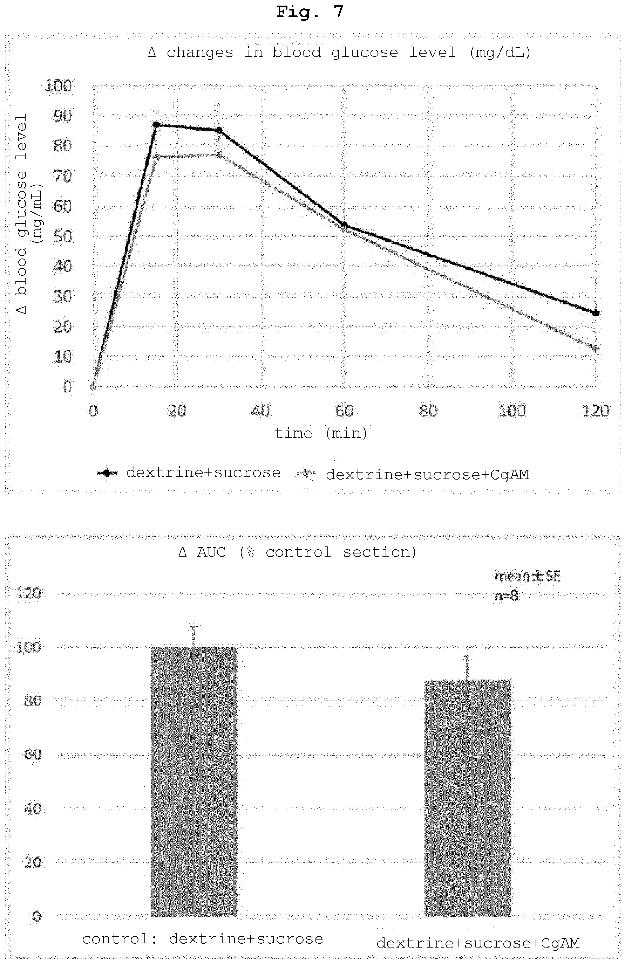

The test results are shown in Fig. 7. From the test results, it was shown that the Δ blood glucose level AUC at 2 hr after administration was suppressed to a low level, and an increase in the blood glucose level was suppressed by the action of CgAM on the mixture of sucrose and dextrin. This is considered to be because sucrose was polymerized by the effect of adding a sugar chain to sucrose by CgAM and digestion thereof became difficult, as observed in Example 9.

### [Example 11] Property modification of starch by CgAM treatment

### (preparation of test substance)

α non-glutinous rice starch (My Alpha K: Joetsu Starch Co., Ltd.) was weighed by 10 g, and 80 g of milli-Q water was added. The mixture was transferred into a standing pouch (Lami Zip Stand Type: produced by SEISANNIPPONSHA LTD.), sealed with a heat sealer, and starch was completely gelatinized by heating at 100°C for 15 min in a thermostatic tank. During gelatinization, the mixture was stirred so that the starch would not become lumps. The starch paste for the control and CgAM was cooled to 37°C, and the starch paste for TtAM was cooled to 50°C, and 10 mL of CgAM solution or TtAM solution prepared such that the amount of each AM added was 1 U per 1 g of starch was added. Milli-Q water (10 mL) was added to the control. Thereafter, they were immediately sealed with a sealer and placed in a thermostatic tank. The control and CgAM

addition section were stood at 37°C, and the TtAM addition section was stood at 50°C for 60 min to cause enzyme reaction. After the enzyme reaction, they were heated at 100°C for 15 min in a thermostatic tank to deactivate the enzyme. The enzyme-treated starch was cooled to room temperature and frozen at - 80°C.

### (Animal test)

The test was performed according to the method of Example 10.

### (Measurement method of blood glucose level)

The measurement was performed according to the method of Example 10.

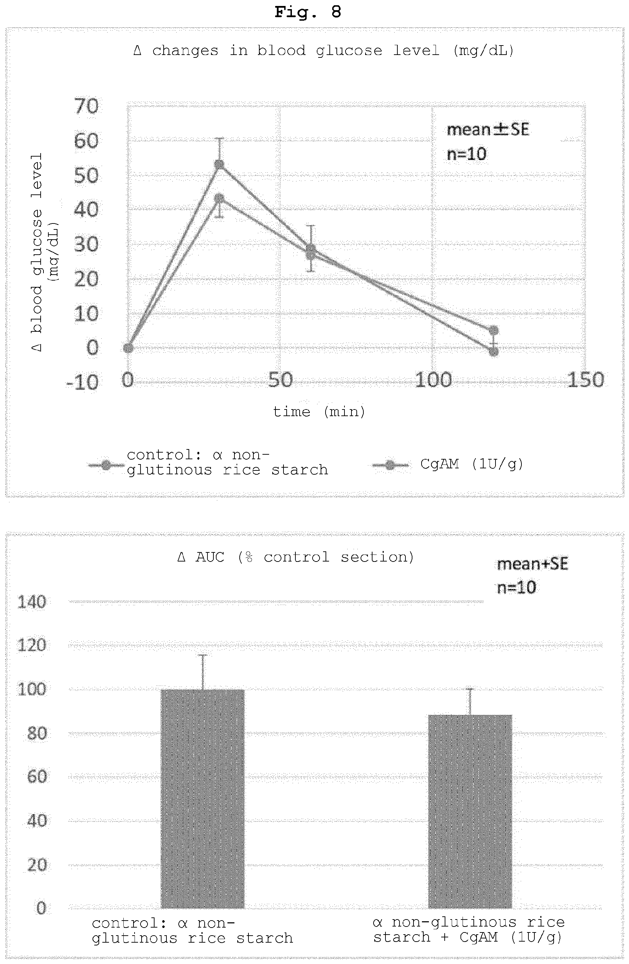

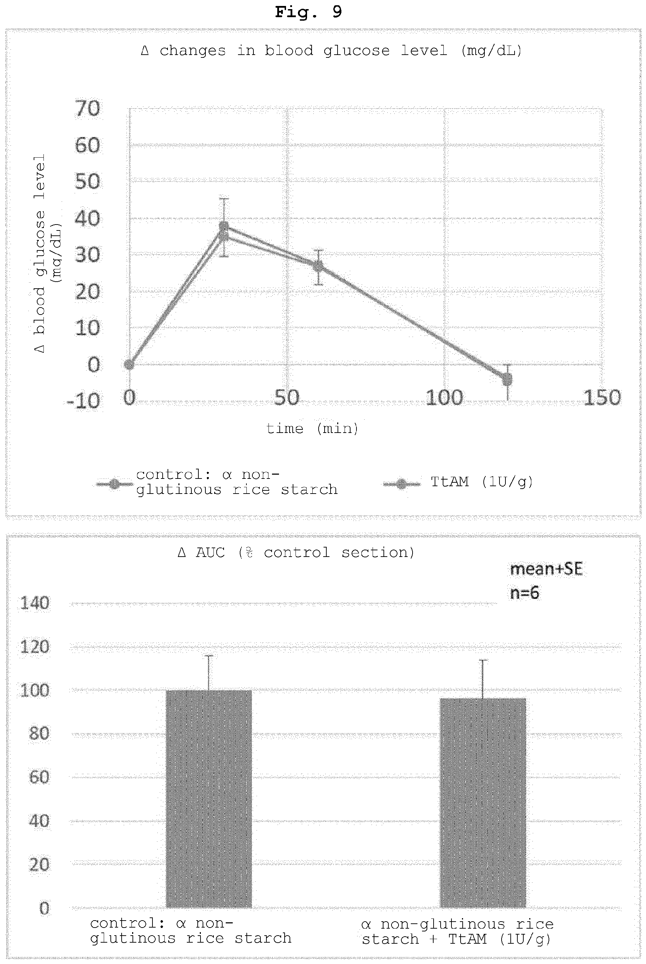

The test results are shown in Figs. 8 and 9. From the test results, it was shown that the Δ blood glucose level AUC at 2 hr after administration was suppressed to a low level, and an increase in the blood glucose level was suppressed in the group administered with CgAM-treated starch, compared with the control group and the TtAM-treated starch administration group.

### [Example 12] Property modification of rice by CgAM treatment

### (Preparation of test substance)

*Hitomebore* from Miyagi prefecture was used as the raw material. For brown rice, the rice harvested on the same day by one manufacturer was ensured, and polished on the same day. The rice was placed in a shading vacuum bag containing an oxygen absorber and kept in a refrigerator at 5°C until the test.

The polished rice was allowed to return to room temperature by 30 min before weighing on the day of cooking rice. The polished rice was weighed using an electronic balance (US6002S, METTLER TOLEDO Co., Ltd.). The polished rice in a sieve was gently stirred clockwise 10 times in tap water in a bowl. The tap water was replaced and the same operation was repeated 5 times. After washing the rice, the rice was picked up in a sieve, transferred into a rice cooking pot, tap water was added so that the water content was 150% on an electronic balance, and CgAM was further added in an amount of 1 U per 1 g of raw rice. After immersing at room temperature for 1 hr, the pot was set in a rice cooker (SR-03GP: Panasonic Corporation) and the rice was cooked. Immediately after cooking rice, the rice cooking pot was turned over on a tray and the cooked rice was taken out. The rice near the pot wall was removed and moved to the end of the bat with a spatula. The cooked rice was flattened with the spatula, wrapped lightly with a small gap on the rice, and then roughly cooled at room temperature for 15 min. The rice was put in UNI-PACK (SEISANNIPPONSHA LTD), smoothed to a thickness of about 2 cm, and frozen in a freezer at -80°C. The next day, the rice was freeze-dried using a freeze-dryer (FDU-2100: TOKYO RIKAKIKAI CO, LTD). After freeze-drying was confirmed, the rice was pulverized using a mixer mill (MM301: Verder Scientific Co. Ltd.). The pulverization sample was dispensed into a standing pouch, tightly sealed, and stored at room temperature.

### (Animal test)

The test was performed according to the method of Example 10. The test substance was suspended in milli-Q water and subjected to the test.

### (Measurement method of blood glucose level)

The measurement was performed according to the method of Example 10. The test substance was administered orally at a total amount of sugar in the test substance of 2 g/20 mL/kg.

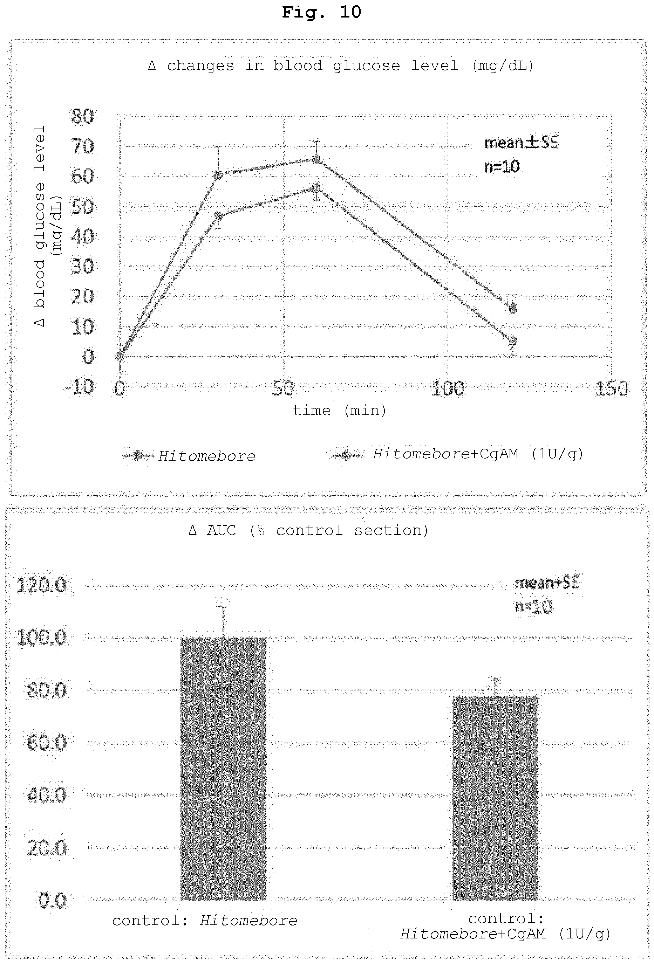

The test results are shown in Fig. 10. It was shown that the Δ blood glucose level AUC at 2 hr after administration was suppressed to a low level, and an increase in the blood glucose level was suppressed in the group administered with CgAM-treated rice, compared with the control group.

### [Industrial Applicability]

According to the present invention, a starch-containing food that has a preferable mouthfeel, is less susceptible to time-related degradation, and is less likely to raise blood glucose level can be produced without the problem possessed by the existing heat-resistant amylomaltase. Therefore, it is highly beneficial in the food producing industry.

This application is based on a patent application No. 2018-058931 filed in Japan (filing date: March 26, 2018), the contents of which are incorporated in full herein.

## Claims

1. A method for producing a starch-containing food, comprising reacting an actinomycete-derived amylomaltase with starch in a raw material.

2. The production method according to claim 1, wherein the actinomycete is the genus Corynebacterium or the genus Streptomyces.

3. The production method according to claim 1 or 2, wherein the actinomycete is selected from the group consisting of Corynebacterium glutamicum, Streptomyces avermitilis, Streptomyces cinnamoneus, Streptomyces griseus, Streptomyces thermoviolaceus, and Streptomyces violaceoruber.

4. The production method according to any one of claims 1 to 3, wherein the starch-containing food is selected from the group consisting of a rice processing food, a wheat processing food, a potato processing food, a corn processing food, a tapioca processing food, and a processing food containing one or more kinds of starch extracted from rice, wheat, potato, corn, or tapioca.

5. The production method according to any one of claims 1 to 4, wherein the starch-containing food comprises sucrose.

6. A method for producing a starch-containing food product, comprising reacting an actinomycete-derived amylomaltase with starch in a raw material, wherein the amylomaltase has the amino acid sequence shown in SEQ ID NO: 1, 3, 4, 5, or 6, or an amino acid sequence not less than 90% identical to the amino acid sequence.

7. A method for modifying property of a starch-containing food, comprising reacting an actinomycete-derived amylomaltase with starch in a raw material.

8. The method according to claim 7, wherein the actinomycete is the genus Corynebacterium or the genus Streptomyces.

9. The method according to claim 7 or 8, wherein the actinomycete is selected from the group consisting of Corynebacterium glutamicum, Streptomyces avermitilis, Streptomyces cinnamoneus, Streptomyces griseus, Streptomyces thermoviolaceus, and Streptomyces violaceoruber.

10. The production method according to any one of claims 7 to 9, wherein the starch-containing food is selected from the group consisting of a rice processing food, a wheat processing food, a potato processing food, a corn processing food, a tapioca processing food, and a processing food containing one or more kinds of starch extracted from rice, wheat, potato, corn, or tapioca.

11. The production method according to any one of claims 7 to 10, wherein the starch-containing food comprises sucrose.

12. A method for modifying property of a starch-containing food product, comprising reacting an actinomycete-derived amylomaltase with starch in a raw material, wherein the amylomaltase has the amino acid sequence shown in SEQ ID NO: 1, 3, 4, 5, or 6, or an amino acid sequence not less than 90% identical to the amino acid sequence.

13. An agent for modifying property of a starch-containing food, comprising an actinomycete-derived amylomaltase.

14. The agent according to claim 13, wherein the actinomycete is the genus Corynebacterium or the genus Streptomyces.

15. The agent according to claim 13 or 14, wherein the actinomycete is selected from the group consisting of Corynebacterium glutamicum, Streptomyces avermitilis, Streptomyces cinnamoneus, Streptomyces griseus, Streptomyces thermoviolaceus, and Streptomyces violaceoruber.

16. The agent according to any one of claims 13 to 15, wherein the starch-containing food is selected from the group consisting of a rice processing food, a wheat processing food, a potato processing food, a corn processing food, a tapioca processing food, and a processing food containing one or more kinds of starch extracted from rice, wheat, potato, corn, or tapioca.

17. The agent according to any one of claims 13 to 16, wherein the starch-containing food comprises sucrose.

18. An agent for modifying property of a starch-containing food, comprising an amylomaltase having the amino acid sequence shown in SEQ ID NO: 1, 3, 4, 5, or 6, or an amino acid sequence not less than 90% identical to the amino acid sequence.
